# EUROPEAN PATENT SPECIFICATION

(11) **EP 3 257 548 B1**
(45) Date of publication and mention of the grant of the patent: **02.08.2023**
(21) Application number: 17171599.8
(22) Date of filing: 17.05.2017
(51) Int. Cl.: A61M 35/00

(54) **FACE SOAKING DEVICE**
GESICHTSEINWEICHVORRICHTUNG
DISPOSITIF DE TREMPAGE DE VISAGE

(30) Priority: 17.06.2016 US 201662351830 P; 28.04.2017 US 201715581919
(43) Date of publication of application: 20.12.2017
(73) Proprietor: Taylor, John Richard, Tyler, TX 75713 (US)
(72) Inventor: Taylor, John Richard, Tyler, TX 75713 (US)
(74) Representative: Barker Brettell LLP

(56) References cited:
- EP-A1- 2 868 218
- US-A1- 2016 096 042
- US-B1- 6 328 031
- US-B1- 7 448 093

## Description

### PRIORITY NOTICE

The present application claims priority to U.S. provisional patent application, application number 62/351,830 filed on June 17, 2016, and U.S. provisional patent application, application number 15/581,919 filed on April 28, 2017.

### CROSS REFERENCE TO RELATED PATENT APPLICATIONS

The present application is related to filed patent application, application number PCT/US15/54576, filed on October 7, 2015.

### TECHNICAL FIELD OF THE INVENTION

The present invention relates in general to skin soaking devices and more specifically to face soaking devices.

### COPYRIGHT AND TRADEMARK NOTICE

A portion of the disclosure of this patent application may contain material that is subject to copyright protection. The owner has no objection to the facsimile reproduction by anyone of the patent document or the patent disclosure, as it appears in the Patent and Trademark Office patent file or records, but otherwise reserves all copyrights whatsoever.

Certain marks referenced herein may be common law or registered trademarks of third parties affiliated or unaffiliated with the applicant or the assignee. Use of these marks is by way of example and should not be construed as descriptive or to limit the scope of this invention to material associated only with such marks.

### BACKGROUND OF THE INVENTION

The skin (epidermis) of humans (and of terrestrial vertebrates) may suffer from a number of problems, such as: acne; wrinkles, including age spots; infections; physical damage; various rashes, including pityriasis rosea, acne rosacea; and the like. Each of these skin problems may be briefly discussed below.

Acne may result from clogged skin pores, which may be visible as pustules or pimples - *i.e.,* what are commonly called blackheads and whiteheads. Such visible acne may be both visually unpleasant and painful. Severe acne may also result in scaring from the physical damage associated with ruptures of follicle walls, which may also form deep cysts under the skin. The clogged skin pores visible as acne may result from an overproduction of sebum oil, keratin, and/or metabolic byproducts of skin pore bacteria, as well as from the cells of skin pore bacteria. A common skin pore bacterium is *Propionibacterium acnes* (*P. acne*)*.*

Undesirable wrinkles on the skin may result from age, environmental factors, genetic factors, and repeated facial expressions. Age may be a factor in wrinkle formation because as skin ages, it may lose elasticity, in part due to accumulated gravitational pull over time and changes in connective tissues. Additionally with age, sebum production may slow (from the sebaceous glands), which may contribute to skin dryness with age, wherein such skin dryness may enhance visibility of wrinkles. Environmental factors may include sun and wind exposure as well as exposure to smoke, which over time may also contribute to wrinkles. Further, consistent facial expressions over time such as squinting, smiling, and even thinking can result in skin wrinkles. And in addition to wrinkles, age spots, such as liver spots and solar lentigines may also appear on the skin as the skin ages and is exposed to various environmental factors over time.

Additionally, various microorganisms, which may include bacteria, fungi, protozoans, and even some small invertebrates may infect skin, both on the surface and within the skin tissue, with varying levels of severity. For example, the mere presence of some such microorganisms, whether dead or alive, may act as an irritant, causing inflammation. Some microorganism metabolic byproducts may also act as irritants; whereas, some byproducts may actually be toxic. And some microorganisms may actually feed on the skin itself and/or the natural secretions of the skin, such as sebum. Such microorganisms may also infect open wounds on the skin and use such open wounds to gain entry to the body, and pose a larger bodily infectious threat.

Additionally, viruses may cause contagious, painful, and/or unpleasant looking lesions and blisters, *e.g.,* cold sores. Such lesions and blisters if ruptured may result in physical damage to the skin, as well as pain. Such viruses may include herpes and herpes like viruses.

With respect to physical damage to the skin, this may include: various wounds, cuts, abrasions, burns, lesions, blisters, ruptures, and the like. Such physical damage to the skin may result in scarring as the skin heals and prior to healing may increase chances for various microorganism infection.

Such skin problems, particularly when occurring on the face, because of the inherent visibility to others of the face, may result in collateral detrimental effects, such as to one's psychological, social, and occupational wellbeing.

Pityriasis rosea may be a type of skin rash. Often, pityriasis rosea may begin with a single "herald patch" an oval red lesion of 2 to 10 centimeters (cm), followed in one or two weeks by a generalized body rash of many small (5 to 10 millimeter (mm)) patches of pink and/or red, flaky, oval shaped lesions, which often appear on the torso, but may also appear on the cheeks and/or at the hairline.

Acne rosacea or just rosacea may be a chronic skin rash condition characterized by facial erythema (redness) and sometimes pimples. Rosacea may affect all ages. Rosacea may typically begin as redness on the central face across the cheeks, nose, or forehead, but may also affect the neck, chest, ears, and/or scalp. In some cases, additional signs, such as semi-permanent redness, telangiectasia (dilation of superficial blood vessels on the face), red domed papules (small bumps) and pustules, red gritty eyes, burning and stinging sensations, and in some advanced cases, a red lobulated nose (rhinophyma), may be present.

The prior state of the art has responded to such problems with a diversity of technologies. For example, there may be a plethora of various topical ointments and creams for treating various skin problems. However, relevant here, may be the application of soaking the affected skin in an immersion liquid. Regardless of explanation, the prior state of art has shown a positive correlation with improvements to the above noted skin problems with soaking the skin in an appropriate immersion liquid. For example, such a treatment modality may be known in the art generally as hydrotherapy when the immersion liquid in question may be predominantly water. However, such hydrotherapy principles may be applied to other such immersion liquids, such as various oils, various paraffin waxes (typically heated), and oil water mixtures (emulsions). As used herein, hydrotherapy may be a means of treating various skin problems, by immersing the skin in a particular immersion liquid, wherein the immersion liquid may be predominantly water or some other liquid, such as an oil in liquid form at room temperature or an appropriate temperature, such as paraffin wax in liquid form when appropriately heated, or an oil and water mixture.

Such hydrotherapy may involve soaking a target region of skin within the immersion liquid. The immersion liquid may comprise various properties. For example, the immersion liquid may contain various dissolved salts, wherein such a liquid may be known herein as a saline solution. For example, the immersion liquid may contain released oxygen, either as dissolved oxygen and/or as gas bubbles within the immersion liquid. For example, the immersion liquid may contain an increased or decreased temperature with respect to room temperature. And for example, the immersion liquid may be directed via one or more jets, such that a stream of liquid pressure may be directed at the target region of skin.

With respect to saline solutions as the immersion liquid, saline and salts as used herein may refer not only to solutions of sodium chloride, but may also refer to other minerals in solution, *e.g*. potassium and/or magnesium, that may be dissolved in a solvent, such as predominantly water. Various negative ions, such as chloride, may also be present in solution with the positive mineral ions. For example, sodium and potassium salt solutions may be present with chloride ions and magnesium may be present with sulfate ions, as in Epsom salt. An immersion liquid using various salts may promote different benefits. For example, some such saline solutions may soften the skin and/or others may tend to moisturize the soaked skin.

Benefits to the skin from soaking the skin in saline solutions may predominantly function by osmosis. Osmosis is a random movement of water molecules across partially-permeable membranes (such as cellular membranes, including skin cells), from an area of high water concentration *(e.g.* within a cell) to an area of low water concentration *(e.g.* the saline solution). Thus osmosis will function to draw water out of cells, including skin cells, when the saline solution has a salinity that is greater than the salinity within the cells. For example, human blood has an average salinity of about 0.85% by weight, which is often rounded to 0.9%. Thus if the saline solution that the skin may be soaking in is greater than 0.9% by weight, there will be osmotic flow of water molecules from the skin cells into the saline solution.

However, it is from this flow of water molecules across cell membranes that several benefits may result for treating and/or improving the various skin problems noted above.

For example, with respect to acne, skin with acne that is exposed to saline solutions may see a reduction in acne. Such reduction may result from the saline solution reducing sebum oil within pores, by the saline solution reducing the population of skin pore bacteria, and/or by the saline solution encouraging a reduction in skin pore size. The saline solution may help to loosen sebum oil from pores. With respect to skin pore bacteria, which may be adapted for non-saline environments, such bacteria may not be adapted to cope with the osmotic flow of water molecules out of the bacterial cells. Such saline solutions may hinder reproduction of such bacterial cells. Such saline solutions may actually kill such bacterial cells. With respect to the reduction in skin pore size, this may also result from osmotic flow of water molecules.

With respect to a reduction in wrinkles, the saline solution may reduce wrinkles by softening the wrinkled skin tissue and by stimulating the sebaceous glands to produce sebum oil which may combat age associated skin dryness. For example, exposing a face to warm water may soften facial skin in preparation and aiding in shaving whiskers (stubble) from that face. Additionally, depending upon the salinity of the given saline solution, the saline solution may have a hydrating effect upon the immersed skin.

With respect to mitigating against microorganism infection of the skin, as noted above, those microorganisms which may be predominantly present on the skin are not typically adapted to withstand osmotic flow of water molecules from within the bacterial cells. Immersion of skin in such saline solutions may result in microorganism population reduction.

With respect to improving a rate of healing damaged skin, skin immersed into saline solutions may experience an improved rate of healing by reducing the populations of microorganisms which may interfere with healing. And the osmotic flow may also aid healing damaged skin by aiding transport of nutrients and repair proteins from within the cells and tissues below the surface skin to the damaged skin site.

Thus immersion of skin into a saline solution which may have a salinity greater than the skin tissue being immersed, may result in a plurality of benefits to the immersed skin.

Now turning to oxygen treatments for the skin and how oxygen may reduce some of the skin problems identified above. Again, regardless of explanation, the state of the prior art shows a positive correlation with exposing skin to oxygen and improvements in the skin.

Molecular oxygen (atmospheric oxygen), *i.e.* O₂, may be essential for cellular respiration and the basis for how each vertebrate cell derives energy via the Krebs Cycle (Citric Acid Cycle). Without a sufficient supply of consistent oxygen to any vertebrate cell, that cell may be hypoxic and may have a diminished capacity to operate normal cellular activities, including a diminished capacity to reproduce, to fight infection, and/or to heal. By providing oxygen in sufficient concentration directly to the skin, such exposed skin may obtain some of its needed oxygen directly, instead of relying largely upon delivery of oxygen via hemoglobin in red blood cells. Such skin cells having a steady available source of oxygen may allow such skin cells a full range of normal cellular activities. Additionally, immune system cells (*e.g*. macrophages and phagocytes) which target and kill infectious microorganisms better perform when such cells have an adequate supply of oxygen. And a second mechanism of oxygen reducing infectious microorganism population may be by oxygen's oxidation properties and ability to form reactive oxygen species that may then oxidize bacterial cellular machinery, such as interfering with bacterial cell walls.

Now oxygen may be applied to the skin in gaseous form and/or released as a dissolved gas and/or as gas bubbles within a liquid, including the immersion liquid. For example, atmospheric air will contain atmospheric oxygen, *e.g.* at approximately 20.95%. A delivered concentration of gaseous oxygen may be increased over the atmospheric percentage by using pure oxygen as a supply source. However, use of gaseous oxygen directed at skin may have the drawback of being difficult to control and manipulate due to the gasses' inherent ability to more freely and disperse. Whereas, release of oxygen in a liquid may provide for better control as the target area of skin may be immersed in the liquid, which then may have oxygen from air or pure oxygen released into the liquid.

The benefits of oxygen and saline solutions may be combined into the same immersion liquid. For example, air (which includes oxygen) and/or oxygen may be pumped or released into an appropriate saline solution. Additionally, such an oxygenated saline solution may be combined with the benefits of controlling a temperature of the oxygenated saline solution.

For example, increasing a temperature of the immersion liquid above room temperature but less than a temperature which may be harmful (*e.g*. painful), allows for an increase in chemical reactions (kinetics). Thus increasing the immersion liquids temperature in such a range will tend to increase the effectiveness of saline solutions as well as the effectiveness of oxygenation of the skin. Additionally, such increased temperature of the immersion liquid may result in an environment that may be soothing and relaxing to a user. Such a soothing and relaxing result may then release stress and mitigate against headaches. Release of stress may promote lowering of blood pressure, healing of damaged skin, and a stronger immune system. Thus, increasing the temperature of the immersion liquid not only may provide direct improvements to how the saline and the oxygen functions to improve the skin, but by creating the soothing and relaxing environment, a collateral benefit of stress release may be achieved, which may also then include a cascade of additional benefits.

Further, increasing the immersion liquids temperature above room temperature may then permit the immersion liquid to be used for heat therapy. Heat therapy may be used to treat not only skin problems, but also other ailments, such as, but not limited to, arthritis, osteoarthritis, fibromyalgia, joint stiffness, bursitis, tendonitis, sprains and pulled muscles. The heat and immersion liquid which may convey the heat, may increase blood flow, improve joint stiffness and reduce pain. For example, heated paraffin waxes as the immersion liquid may be utilized. Such heated paraffin wax may soften hardened skin caused by scleroderma, a disease in which collagen accumulates on the body.

In addition or alternatively, decreasing the immersion liquids temperature below room temperature may then permit the immersion liquid to be used for cold therapy. Chilling the liquid by use of a chiller, chilling equipment, and/or by introduction of ice, may then permit various cold therapies to be used to treat the face or other body part which may be removably immersed into the chilled liquid. Additionally or alternatively, heat therapy may be alternated with cold therapy; wherein such alternation of warmth and cold may aid in increasing blood flow, facilitating removal of cellular toxins (*e.g*., but not limited to, lactic acid), and/or promoting healing of burned or traumatized tissue.

Additionally, liquid jets, for example water jets, when directed at the immersed skin may also result in an environment that is soothing and relaxing to the user. Such water jets also may have their benefit increased when the immersion temperature is increased as noted per above.

Light therapy may also be used to impart various benefits to the exposed skin and/or body in general. Light therapy may involve directing a source of light at skin. Some wavelengths of light have found to increase healing rates of damaged skin, such damaging including cuts, scrapes, bruising, lacerations, lesions, and the like. Light such as ultraviolet (UV) light may also be used for skin tanning purposes. However, both existing oxygen therapy and existing light therapy are conducted in a treatment environment of atmospheric air, *i.e.,* not with an article to be treated *(e.g.* a region of skin) submerged within an immersion liquid.

Additionally, it may be desirable to expand beyond just oxygen, air, or air enriched with oxygen, as treatment gasses for skin.

Additionally, current light therapy devices generally are directed at emitting only a very narrow range of wavelengths, generally within the visible light spectrum, near infrared (IR), and near ultraviolet (UV). It would be desirable to have expanded devices that may be capable of emitting electromagnetic (EM) radiation in various wavelengths that may encompass regions of the entire EM spectrum, *i.e.* not necessarily a single device capable of emitting across the entire EM spectrum (since different technologies may be required to produce a given range of wavelengths), but rather a multitude of EM emitting devices where each different device may be capable of emitting a particular range of wavelengths, such that these different EM emitting devices may collectively be able to cover the entire EM spectrum.

Conducting oxygen therapy and/or light therapy or other EM therapy within the immersion liquid may be desirable for several reasons. Because the liquid is more dense than atmospheric air, more control over directing oxygen (or other gas) to a target region on the article (*e.g*., immersed skin region) may be achieved over conducting oxygen therapy in atmospheric air, where expelled oxygen quickly dissipates into the atmospheric air. By using the immersion liquid to removably submerge the target region of the article, useful properties of the liquid may be tailored for specific applications with respect to the target region of the article. For example, liquid water, such as saline solutions, may soften the skin and make such softened skin better able to benefit from exposure to oxygen and/or various wavelengths of light. The additives in the liquid may be used to heal, cleanse, rejuvenate, sanitize, sterilize, and the like. Likewise, controlling a temperature of the liquid may then be able to impart heat or withdraw heat from the target region of the article in a much greater efficiency than may be possible where the treatment environment is atmospheric air and not the liquid. Additionally, controlling the temperature (up, down, or maintaining) of the liquid may increase or decrease the efficacy of the additives, *e.g*., from a kinetics perspective.

Furthermore, it has been discovered that conducting light therapy or other EM therapy may be enhanced when the EM radiation may be emitted through a plurality of bubbles within the immersion liquid, by providing an increased coverage of the target region of the article receiving EM radiation in comparison to if there were no bubbles. The emitted EM radiation and the bubbles produce an optical chain reaction (OCR) phenomena that provides this enhancement.

However, as noted above, with respect to such skin problems on the face, these problems are exacerbated because the high visibility of the face. Additionally, these skin problems on the face are exacerbated because the current state of the art does not provide a means by which the user may immerse the face to receive hydrotherapy, wherein the hydrotherapy immersion liquid may comprise saline solutions, delivery of oxygen (and/or other gasses), heating means for increasing and/or decreasing immersion liquid temperature, and/or use of liquid jets. The problem that the prior state of the art has failed to address, until this invention, results from two biological facts. One, terrestrial vertebrates breathe from their nose and/or mouth located on the face and thus a hydrotherapy means for the face needs to provide a means by which the user may breathe while the user's face is immersed. Otherwise immersion of the face is limited to how long the user can hold their breath. And two, all pre-existing vessels have no means to accommodate a neck region of the user, particularly the soft tissue regions of the neck (front and sides of the neck), so if the user were to submerge the user's face into a pre-existing vessel, a rim of that vessel would press into the neck region causing discomfort rendering the prior state of the art ineffective for hydrotherapy of the face. Or the user would have to angle their head into the prior art vessel and attempt to hold their head at an uncomfortable angle to soak their face, which if is prolonged may result in neck pain. Additionally, it may be desirable if such a device might, in at least some embodiments, comfortably support the head region of the user, particularly the forehead, to promote facial immersion that may be comfortable and not strain the neck; wherein the user may soak their face, in comfort, for extended periods of time.

There is a need in the art for devices and/or methods that permit treating specifically targeted regions of the articles (e.g., skin) to be removably submerged in the immersion liquid and then treated with various gas bubbles, such as oxygen, treated with various wavelengths of EM radiation, such as visible light, and/or providing for an enhanced EM radiation coverage of the treated region by a synergistic combination of EM radiation and bubbles that may result from directing EM radiation through bubbles in the liquid.

US2016/096042 discloses face soaking devices and methods of use. The device may comprise a vessel, a vessel neck gasket, and a breathing apparatus. The vessel may be configured to hold a liquid to submerge a face of a user or a portion thereof. US7448093 discloses a safety suit material that can be connected to a plurality of gloves or boots to provide a gas or liquid-proof connection. US6328031 discloses an apparatus having a face mask removably attachable to a firefighting hood. EP2868218 discloses a sealing device for garments to sealingly connect an annular layer and a wearable layer to the sealing device.

There then is a need in the art for a device which may promote comfortable face immersion into the immersion liquid that both allows the user to breathe while the face is immersed and that may be comfortable to the neck of the user.

It is to these ends that the present invention has been developed.

### BRIEF SUMMARY OF THE INVENTION

To minimize the limitations in the prior art, and to minimize other limitations that will be apparent upon reading and understanding the present specification, one or more embodiments may describe a face soaking device.

Some embodiments may provide for a device which may be used to place and hold a person's face into a liquid for an extended time while the person conveniently and/or comfortably breathes through a breathing tube *(e.g.,* a breathing apparatus). While the person has his or her face immersed in the liquid, the device may aerate the liquid (with various gasses). In some embodiments, the device may be designed to minimize or prevent the spillage of liquid onto the person's clothing or the immediate area around the device.

In some embodiments the face soaking device may comprise a vessel and a vessel neck gasket. The vessel may be configured to hold a liquid to submerge a face of a user or a portion thereof. The vessel neck gasket may be removably joined to the vessel. The vessel neck gasket may be configured to comfortably accommodate a portion of the user's neck. In some embodiments, the face soaking device may also comprise a breathing apparatus that may be in removable contact with: the vessel, with a head support, and/or with the user. The breathing apparatus may be configured to permit the user to breathe while the user's face (in whole or in part) may be submerged within the liquid. When the vessel may be filled with the liquid to at least a sufficient level, the user may soak the face or the portion thereof, such that skin being soaked receives a benefit.

It is an objective of the present invention to provide a face soaking device that may be used to reduce severity of facial acne by immersing the face within the immersion liquid. The device according to the invention is defined in claim 1. Further embodiments of the invention are defined in dependent claims.

It is another objective to provide the face soaking device that may be used to reduce severity of facial wrinkles and/or facial age spots by immersing the face within the immersion liquid.

It is another objective to provide the face soaking device that may be used to reduce severity of microorganism infection, including, but not limited to viral, bacterial, and/or fungal infections, of facial skin by immersing the face within the immersion liquid.

It is another objective to provide the face soaking device that may be used to reduce severity of physical damage to facial skin by immersing the face within the immersion liquid.

It is another objective to provide the face soaking device that may permit a user to submerge the user's face within the immersion liquid by the face soaking device comprising a vessel which may be configured to hold the immersion liquid.

It is another objective to provide the face soaking device that may permit the user to breath while the user's face may be immersed in the immersion liquid.

It is another objective to provide the face soaking device that may permit the user to immerse the user's face within the immersion liquid while maintaining comfort to the neck where the neck may contact the face soaking device, particularly where the soft tissue of the neck may contact the face soaking device.

It is another objective to provide the face soaking device that may minimize immersion liquid spillage around the user's neck when the user's face may be immersed within the immersion liquid.

It is another objective to provide the face soaking device that may catch spilled immersion liquid from a main vessel *(e.g.,* the vessel) holding the immersion liquid.

It is another objective to provide the face soaking device that may permit the user to immerse the user's face within the immersion liquid while maintaining comfort to the neck and mitigating against neck strain, by supporting a portion of the user's head that may be within the vessel, particularly that of the forehead.

It is another objective to provide the face soaking device, wherein a head support *(e.g.,* a head rest subassembly) may be adjustable; wherein such adjustments may be in a vertical direction (height direction) and/or in a forwards-backwards direction.

It is another objective to provide the face soaking device wherein the immersion liquid may receive various gasses into the immersion liquid.

It is another objective to provide the face soaking device wherein the immersion liquid may be oxygenated by a release of air and/or oxygen within the immersion liquid.

It is another objective to provide the face soaking device wherein a temperature of the immersion liquid may be increased or decreased with respect to a room temperature of the face soaking device.

It is another objective to provide the face soaking device wherein the vessel may be insulated to help control the temperature of the immersion liquid.

It is another objective to provide the face soaking device wherein the face soaking device may be used for heat therapy and/or for cold therapy.

It is another objective to provide the face soaking device wherein the face soaking device may be used for heat therapy and/or for cold therapy, wherein the heat therapy and/or the cold therapy may be used to treat not only skin problems, but also other ailments, such as, but not limited to, arthritis, osteoarthritis, fibromyalgia, joint stiffness, bursitis, tendonitis, sprains, pulled muscles, and the like.

It is another objective to provide the face soaking device wherein the face soaking device may be used for heat therapy and/or for cold therapy, wherein the heat therapy and/or the cold therapy may increase blood flow, improve joint stiffness, reduce pain, and the like.

It is another to provide the face soaking device wherein the face soaking device may be used for cold therapy, wherein the cold therapy may increase blood flow, improve joint stiffness, reduce pain, reduce swelling, and the like.

It is another objective to provide the face soaking device wherein the face soaking device may be used for alternating between heat therapy and cold therapy, wherein the alternating heat and cold therapy may increase blood flow, improve joint stiffness, reduce pain, reduce swelling, improve healing, aid in removing cellular toxins, and the like.

It is another objective to provide the face soaking device wherein the face soaking device may be used for soaking facial skin for at least a purpose of softening such facial skin and/or for softening facial hair (*e.g*., whiskers and/or stubble). For example, such skin softening may be beneficial for facial shaving.

It is another objective to provide the face soaking device wherein the face soaking device may be used for treating burns, external and/or internal.

It is another objective to provide the face soaking device wherein the face soaking device may be used for lightening skin shading.

It is another objective to provide the face soaking device wherein the face soaking device may be used for darkening skin shading *(e.g.,* skin tone or skin hue).

It is another objective to provide the face soaking device wherein the immersion liquid may be paraffin wax.

It is another objective to provide the face soaking device wherein interior surface of the vessel may be smooth to facilitate draining of the immersion liquid and to facilitate cleaning and sanitation of the face soaking device.

It is another objective to provide the face soaking device that may be portable and that may be carried by a single adult user.

It is another objective to provide the face soaking device wherein interior surfaces of the vessel may comprise one or more jet nozzles, one or more intakes, and a means for pumping the immersion liquid from the intakes and through the jet nozzles such that a pressure of immersion liquid may be directed to portions of the immersed face.

It is another objective to provide the face soaking device wherein jet nozzle positioning may be adjustable.

It is another objective to provide the face soaking device wherein facial immersion within the immersion liquid within the vessel may be soothing and relaxing to the user, such stress may be reduced.

It is another objective that such a stress releasing use of the face soaking device may result in further collateral benefits such as promoting lowering of blood pressure, mitigation against headache severity, and/or strengthening the user's immune system.

It is yet another objective to provide a face soaking device that may utilize, quality and reliable manufacturing methods, but that may also be efficient and less expensive manufacturing methods.

These and other advantages and features of the present invention are described herein with specificity so as to make the present invention understandable to one of ordinary skill in the art, both with respect to how to practice the present invention and how to make the present invention.

### BRIEF DESCRIPTION OF THE SEVERAL VIEWS OF THE DRAWINGS

Elements in the figures have not necessarily been drawn to scale in order to enhance their clarity and improve understanding of these various elements and embodiments of the invention. Furthermore, elements that are known to be common and well understood to those in the industry are not depicted in order to provide a clear view of the various embodiments of the invention.
**FIG. 1** may depict an embodiment of an overall assembled face soaking device, shown from a top perspective view.
**FIG. 2A** may depict a close up of a front and a top portion of the face soaking device of **FIG. 1****.**
**FIG. 2B** may depict a front view of the face soaking device of **FIG. 2A****.** **FIG. 2B** also includes **sectional-line 7 -7.**
**FIG. 2C** may depict a top view of a front portion of the face soaking device of **FIG. 2A****.**
**FIG. 2D** may depict a back view of a front portion of the face soaking device of **FIG. 2A****.**
**FIG. 3A** may depict a partial exploded view of a front portion of the face soaking device of **FIG. 2A****,** shown from a top front perspective view.
**FIG. 3B** may depict a partial exploded view of a front portion of the face soaking device of **FIG. 2A****,** shown from a top back perspective view.
**FIG. 4A** may depict a close up of a front and a top portion of the face soaking device of **FIG. 2A****,** but with a vessel neck gasket and a clamp removed, so a neck-gasket-accommodator may be visible.
**FIG. 4B** may depict a front view of the face soaking device of **FIG. 4A****.**
**FIG. 4C** may depict a top view of a front portion of the face soaking device of **FIG. 4A****.**
**FIG. 4D** may depict a back view of a front portion of the face soaking device of **FIG. 4A****.**
**FIG. 5A** may depict an exploded view of a vessel neck gasket; wherein a carrier may be separated from a flexible member, shown from a top front perspective view.
**FIG. 5B** may depict the assembled vessel neck gasket of **FIG. 5A****,** shown from a top front perspective view.
**FIG. 5C** may depict the assembled vessel neck gasket of **FIG. 5A****,** shown from a front view.
**FIG. 5D** may depict the assembled vessel neck gasket of **FIG. 5A****,** shown from a back view.
**FIG. 5E** may depict the assembled vessel neck gasket of **FIG. 5A****,** shown from a top view.
**FIG. 5F** may depict the assembled vessel neck gasket of **FIG. 5A****,** shown from a bottom view.
**FIG. 5G** may depict the assembled vessel neck gasket of **FIG. 5A****,** shown from a left side view. (A right side view may be a mirror image of **FIG. 5G****.**)
**FIG. 6A** may depict a clamp, shown from a top front perspective view.
**FIG. 6B** may depict the clamp of **FIG. 6A****,** shown from a front view.
**FIG. 6C** may depict the clamp of **FIG. 6A****,** shown from a back view.
**FIG. 6D** may depict the clamp of **FIG. 6A****,** shown from a top view.
**FIG. 6E** may depict the clamp of **FIG. 6A****,** shown from a bottom view.
**FIG. 6F** may depict the clamp of **FIG. 6A****,** shown from a side view.
**FIG. 6G** may depict a close up of one end (a left end) of the clamp of **FIG. 6A****,** shown from a top front perspective view.
**FIG. 7** may depict a cross-sectional view along **sectional-line 7 -7** that is shown in **FIG. 2B****.**
**FIG. 8** may depict a cross-sectional view of the neck-gasket-accommodator, the vessel neck gasket, and an additional embodiment of the clamp.
**FIG. 9** may depict a cross-sectional view of the neck-gasket-accommodator, the vessel neck gasket, and an additional embodiment of the clamp.
**FIG. 10** may depict a cross-sectional view of the neck-gasket-accommodator, the vessel neck gasket, and an additional embodiment of the clamp.
**FIG. 11** may depict a cross-sectional view of the neck-gasket-accommodator, the vessel neck gasket, and an additional embodiment of the clamp.
**FIG. 12A** may show that a clamp embodiment may resemble a letter "f" in cross-section.
**FIG. 12B** may show that a clamp embodiment may resemble a letter "L" in cross-section.
**FIG. 12C** may show that a clamp embodiment may resemble a letter "J" in cross-section.
**FIG. 12D** may show that a clamp embodiment may resemble a letter "C" in cross-section.
**FIG. 12E** may depict a cross-sectional view of a neck-gasket-accommodator.

### REFERENCE NUMERAL SCHEDULE

- 100: face soaking device **100**
- 120: vessel **120**
- 121: at least one wall **121**
- 122: rim **122**
- 125: at least one base **125**
- 130: breathing apparatus **130**
- 140: head rest subassembly **140**
- 150: heater subassembly **150**
- 160: aerator **160**
- 170: controls **170**
- 400: neck-gasket-accommodator **400**
- 402: contour **402**
- 404: horizontal width **404**
- 406: maximum vertical length **406**
- 408: receiving-channel **408**
- 410: two opposing opening wall-edges **410**
- 412: pocket **412**
- 500: vessel neck gasket **500**
- 501: mating edge **501**
- 503: top edge **503**
- 505: flexible member **505**
- 507: internal surface **507**
- 509: external surface **509**
- 511: carrier **511**
- 600: clamp **600**
- 601: terminal end **601**
- 603: mating-wall-edge **603**
- 605: snap latch **605**
- 800: clamp **800**
- 811: finger-pull **811**
- 813: chamfer **813**
- 815: tab **815**
- 900: clamp **900**
- 911: finger-pull **911**
- 1000: clamp **1000**
- 1011: finger-pull **1011**
- 1100: clamp **1100**

### DETAILED DESCRIPTION OF THE INVENTION

In the following discussion that addresses a number of embodiments and applications of the present invention, reference is made to the accompanying drawings that form a part thereof, where depictions are made, by way of illustration, of specific embodiments in which the invention may be practiced. It is to be understood that other embodiments may be utilized and changes may be made without departing from the scope of the invention.

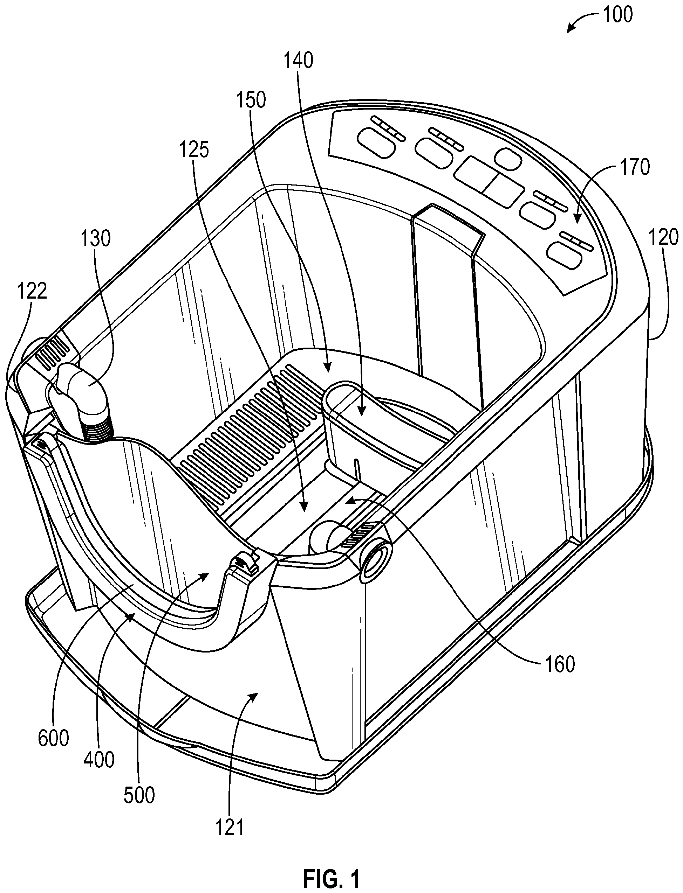

**FIG. 1** may depict an embodiment of an overall assembled face soaking device **100,** shown from a top perspective view. In some exemplary embodiments, as may be depicted in **FIG. 1****,** face soaking device **100** may comprise: a vessel **120,** a vessel neck gasket **500,** and a breathing apparatus **130.**

Continuing discussing **FIG. 1****,** in some embodiments, vessel **120** may be hold a liquid in a sufficient volume to submerge a whole face of a user or a portion thereof. It is obvious to one of ordinary skill in the art, that vessel **120** may be configured to hold the liquid at a sufficient level to permit the user to submerge (immerse) the whole of their face, or a portion thereof within the liquid, while the liquid may be held within vessel **120.** This sufficient level may be a liquid level where when the user inserts their face into an internal volume of vessel **120** and rests a first portion of their neck upon a vessel neck gasket **500,** such that their face may be completely immersed in the liquid. The maximum liquid level of vessel **120** may be greater (higher) than this sufficient level.

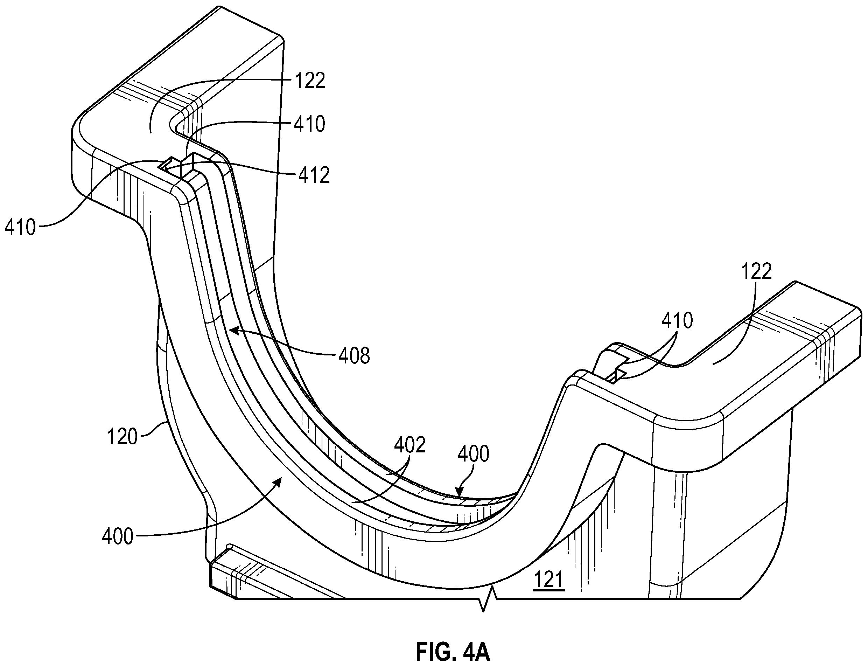

In some embodiments, vessel **120** may comprise at least one wall **121** and at least one base **125.** At least one wall **121** and at least one base **125** may be in physical contact with each other. At least one wall **121** may comprise a neck-gasket-accommodator **400.** (*See e.g.,* **FIG. 4A** for neck-gasket-accommodator **400.)** Neck-gasket-accommodator **400** may be configured to accommodate vessel neck gasket **500.** In some embodiments, neck-gasket-accommodator **400** may be formed as a cutout into a region of at least one wall **121,** from a top of vessel **120.** In some embodiments, neck-gasket-accommodator **400** may be formed as an integral molded structure of a region of at least one wall **121.** Vessel neck gasket **500** may be removably joined to vessel **120.** Where vessel neck gasket **500** may join vessel **120,** *i.e.,* along such surfaces of physical contact, a primary water tight seal may be formed in or at the vicinity of neck-gasket-accommodator **400.** For example, and without limiting the scope of the present invention, in some embodiments, this "vicinity" of neck-gasket-accommodator **400** may be two inches or less from structure of neck-gasket-accommodator **400.** In other embodiments, other distances may be used for the "vicinity" of neck-gasket-accommodator **400.** Vessel neck gasket **500** may receive a first portion of a neck region of the user when the whole face or the first portion thereof may be submerged in the liquid within the internal volume of vessel **120.** The first portion of the neck region of the user may be where their neck physically contacts vessel neck gasket **500.** Note, the nature of the physical contact between the first portion of the neck region of the user and vessel neck gasket **500** may be removable. When vessel neck gasket **500** may receive the first portion of the neck region of the user, a secondary water tight seal may be formed between this first portion of the neck region and vessel neck gasket **500.**

In some embodiments, vessel **120** may be double hulled.

In some embodiments, breathing apparatus **130** may be in physical contact with vessel **120,** as may be shown in **FIG. 1****.** In some embodiments, a breathing apparatus embodiment may be in physical contact with a head rest subassembly. In both such embodiments, the nature of the physical contact between breathing apparatus embodiments (*e.g.*, **130**) and vessel **120;** or between breathing embodiments and the given head rest subassembly, may be removable in some embodiments; while non-removable in other embodiments. In some embodiments, the user may be able to breathe using breathing apparatus **130** when a mouth of the user may be holding a mouth piece of breathing apparatus **130.**

When vessel **120** may be filled with the liquid to a level at or less than a maximum liquid level of vessel **120,** the user may soak their whole face or the portion thereof for a time period. The skin being soaked in the liquid for this time period may receive a health, an aesthetic, and/or a soothing benefit.

In some embodiments, the benefit may comprise one or more of a reduction in acne, a reduction in wrinkle severity, a softening of skin, moisturizing of skin, promotion of relaxation, promotion of healing of damaged skin, promotion of healing of infected skin, reduction in rash severity, reduction and/or elimination of headaches (including migraine), promotion of healing of traumatized tissue (including burned tissue), lightening skin shades, darkening skin shades (tone, hue), reduction in swelling, and the like. Such benefits may derive from facial skin exposure to the liquid where characteristics of the liquid may comprise one or more of the liquid being a saline solution, the liquid being a saline solution with a salt concentration greater than 0.9% by weight, presence of air and/or oxygen within the liquid, temperature of the liquid being less than or greater than ambient room temperature, circulation of the liquid within vessel **120,** and/or the liquid being directed against a portion of the skin in the form of stream or jet of the liquid pressure.

In some embodiments, face soaking device **100** may further comprise a head rest subassembly **140.** In some embodiments, at least some portion of head rest subassembly **140** may be attached to vessel **120.** *See e.g.,* **FIG. 1****.** In some embodiments, the at least some portion of head rest subassembly **140** may be removably attached to vessel **120.** In some embodiments, head rest subassembly **140** may comprise a support member or a strap. In some embodiments, at least some portion of the support member or at least some portion of the strap may be located in the internal volume of vessel **120.** In some embodiments, the support member or the strap physically supports a portion of the head *(e.g.,* a forehead) of the user when the whole face or portion thereof is removably located in the internal volume of vessel **120.** *See e.g.,* **FIG. 1****.**

In some embodiments, face soaking device **100** may further comprise a heater subassembly **150.** In some embodiments, at least a portion of heater subassembly **150** may be attached to vessel **120.** *See e.g.,* **FIG. 1****.** In some embodiments, heater subassembly **150** may comprise a heating element that heats at least a portion of the liquid within the internal volume of vessel **120.** In some embodiments, heater subassembly **150** may provide heating and/or coo ling to the at least the portion of the liquid within the internal volume of vessel **120.**

In some embodiments, face soaking device **100** may further comprise an aerator **160.** *See e.g.,* **FIG. 1****.** In some embodiments, aerator **160** may comprise a gas diffuser and a gas source in physical communication with the gas diffuser. In some embodiments, the gas diffuser may be attached to vessel **120.** In some embodiments, the gas diffuser may be removably attached to vessel **120.** In some embodiments, the gas source provides gas to the gas diffuser. In some embodiments, the gas diffuser releases at least some of the gas received through a porous structure of the gas diffuser into the internal volume of vessel **120;** such that bubbles may be released into at least some of the liquid in the internal volume of vessel **120.**

In some embodiments, face soaking device **100** may further comprise at least one electromagnetic (EM) emitter. In some embodiments, the at least one EM emitter emits electromagnetic radiation at predetermined wavelengths. In some embodiments, at least some portion of the at least one EM emitter may be attached to vessel **120.** In some embodiments, at least some of emitted electromagnetic radiation is emitted into the internal volume of vessel **120.** For example, and without limiting the scope of the present invention, in some embodiments, the at least one EM emitter may comprise at least one light source, such as, but limited to a LED (light emitting diode) or one or more banks of LEDs.

In some embodiments, face soaking device **100** may further comprise controls **170** for controlling electronics and/or electro-mechanical components of face soaking device **100.** Such electronic and electro-mechanical components may comprise one or more: heater subassembly **150,** aerator **160,** and/or the at least one electromagnetic emitter. *See e.g.,* **FIG. 1****.**

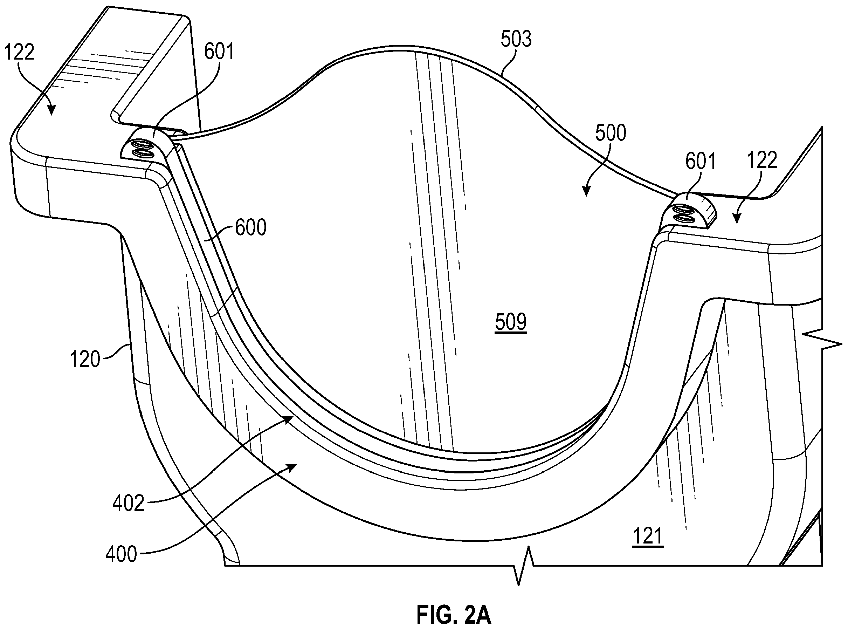

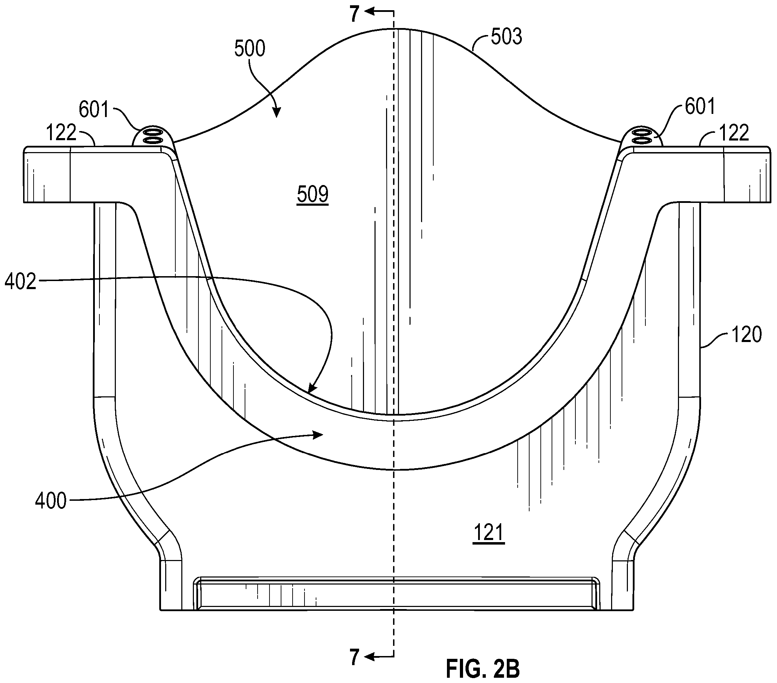

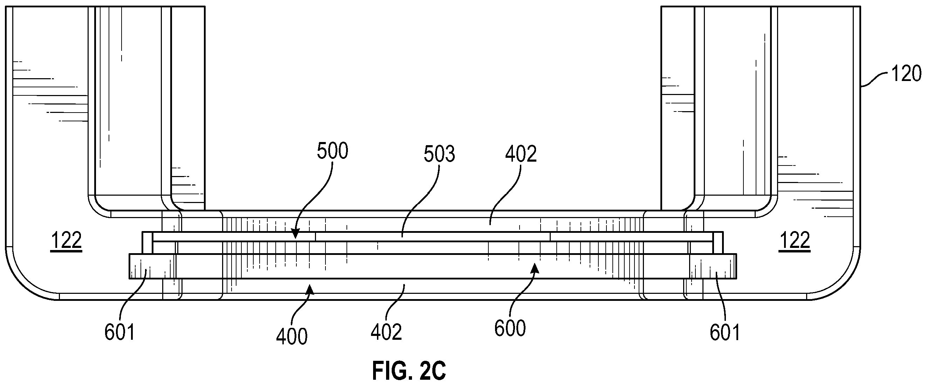

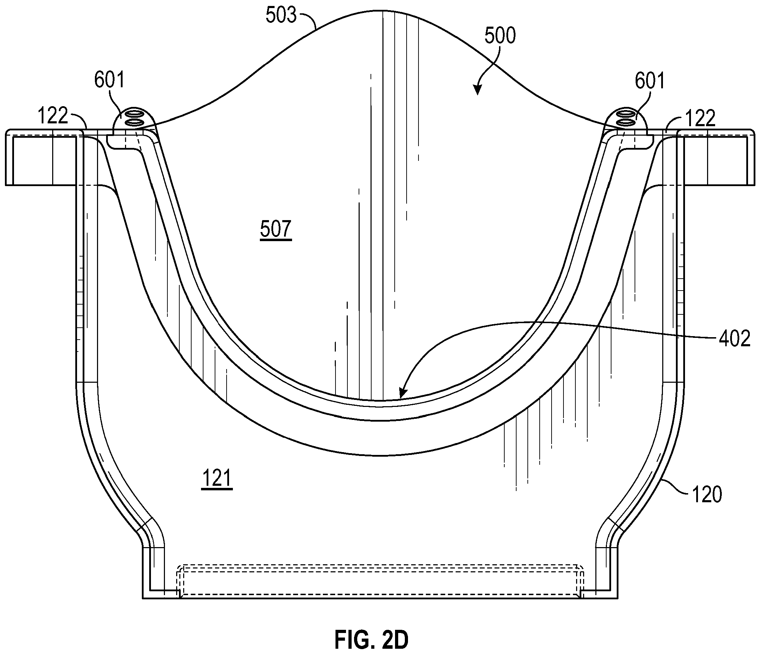

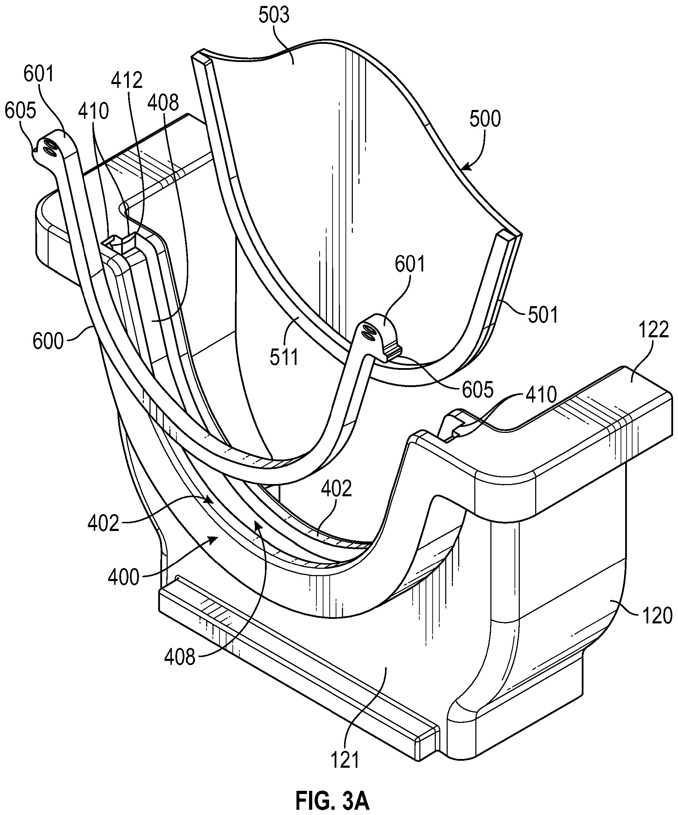

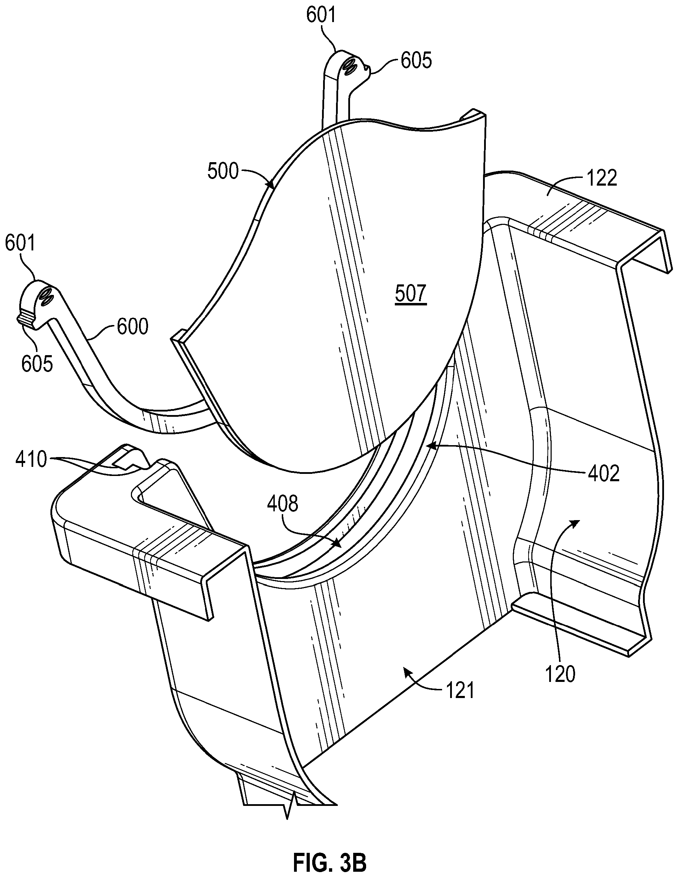

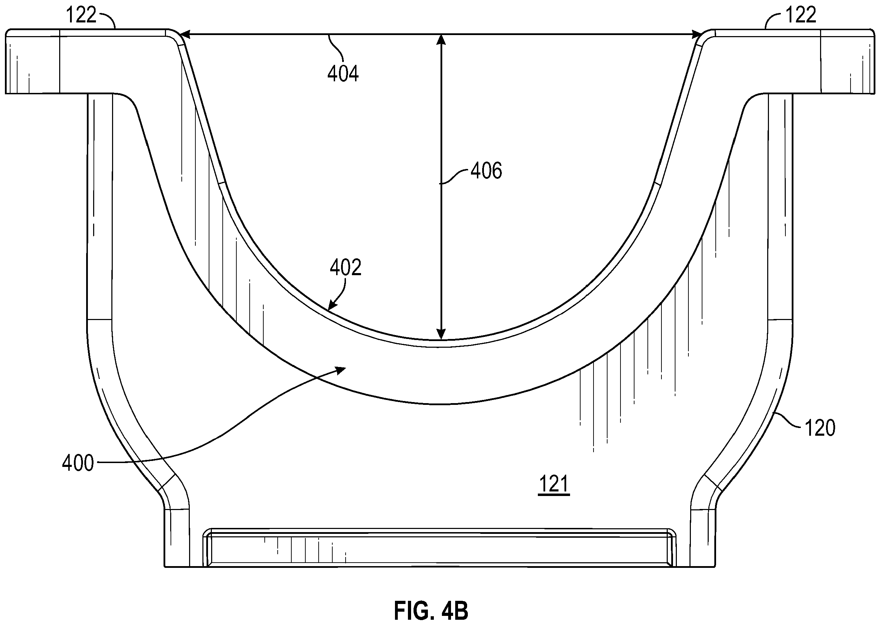

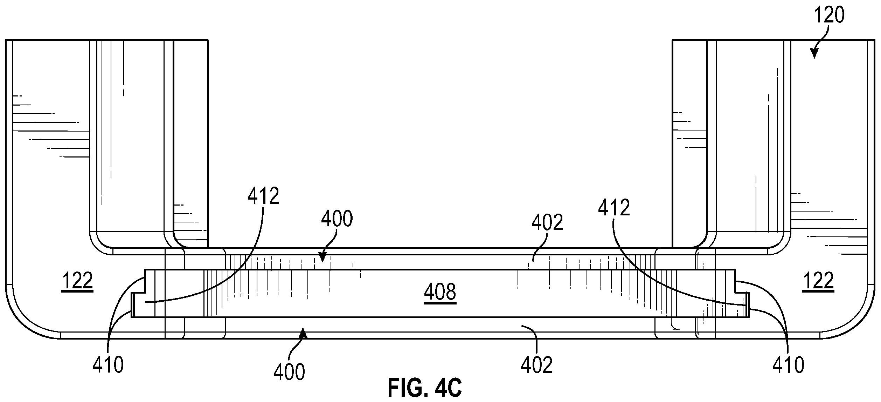

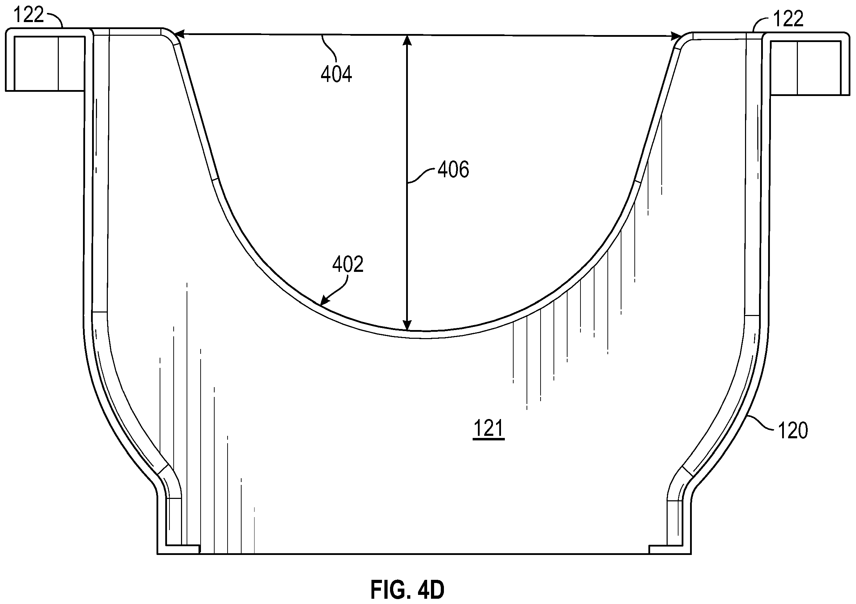

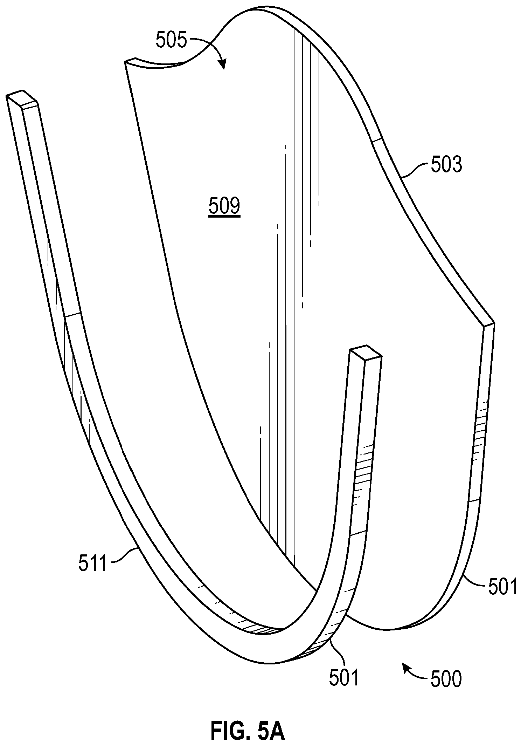

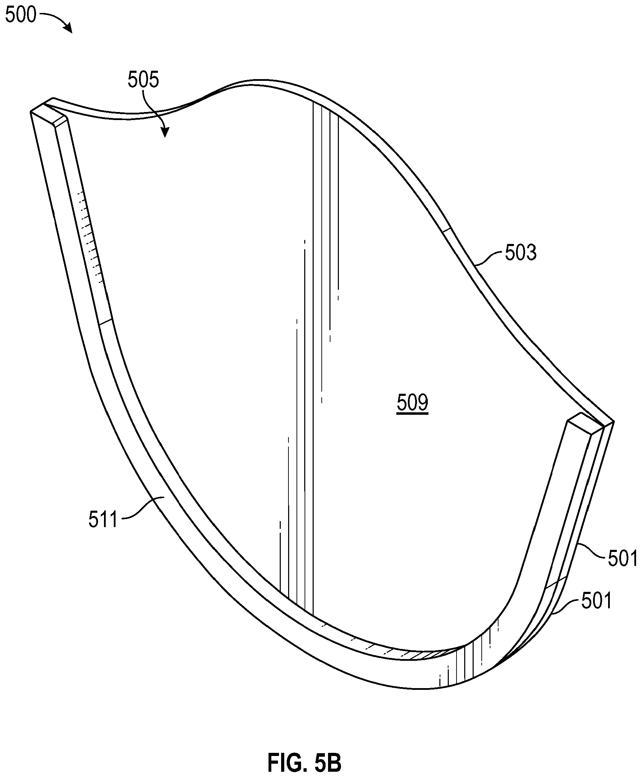

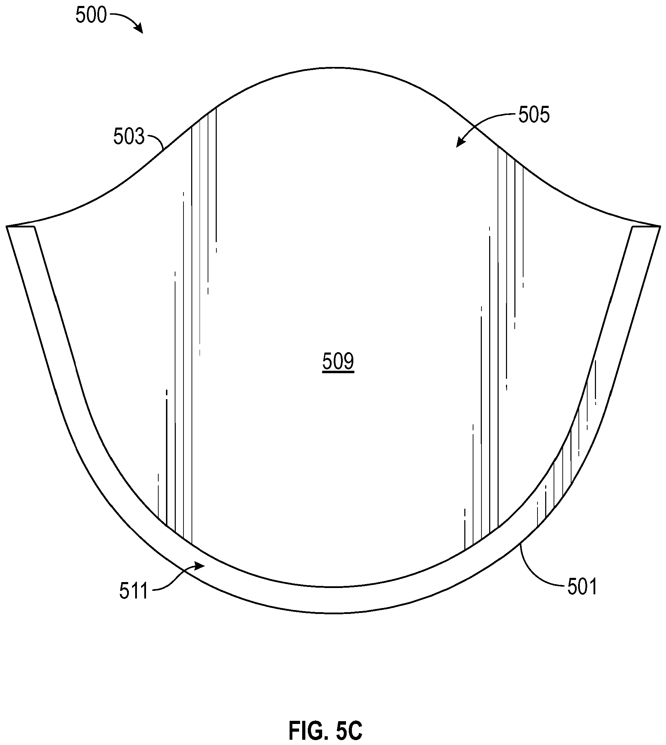

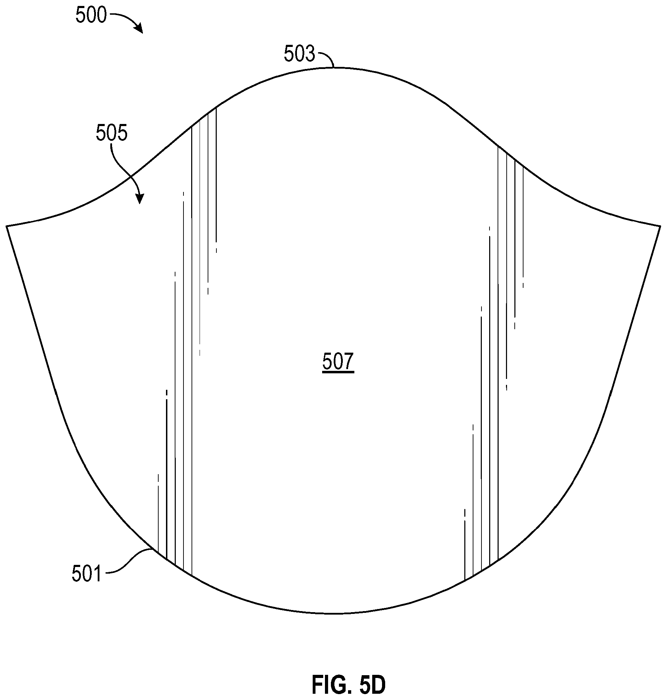

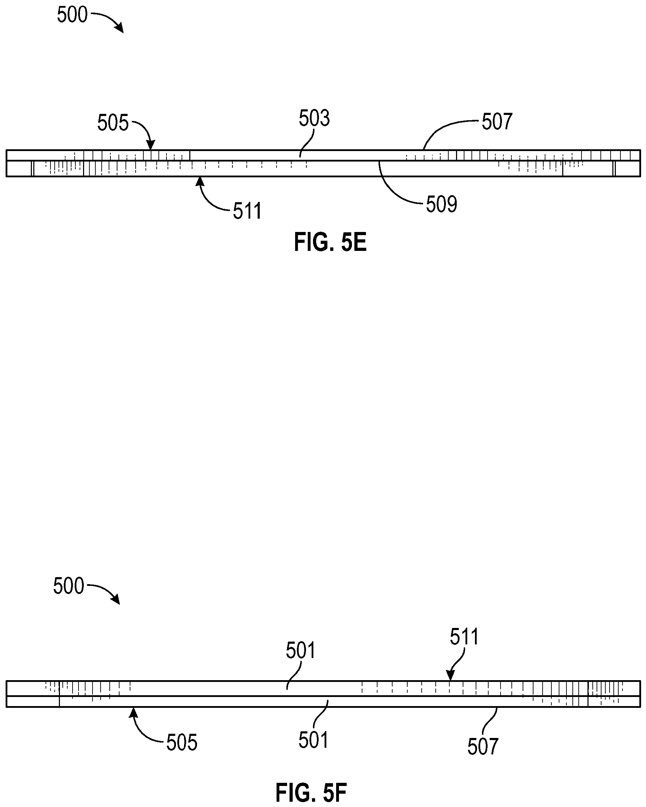

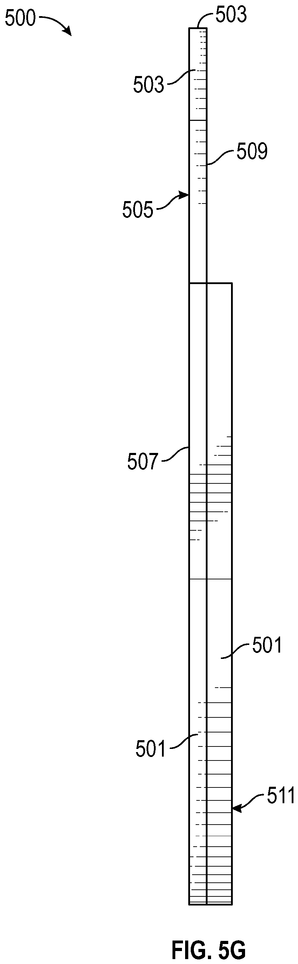

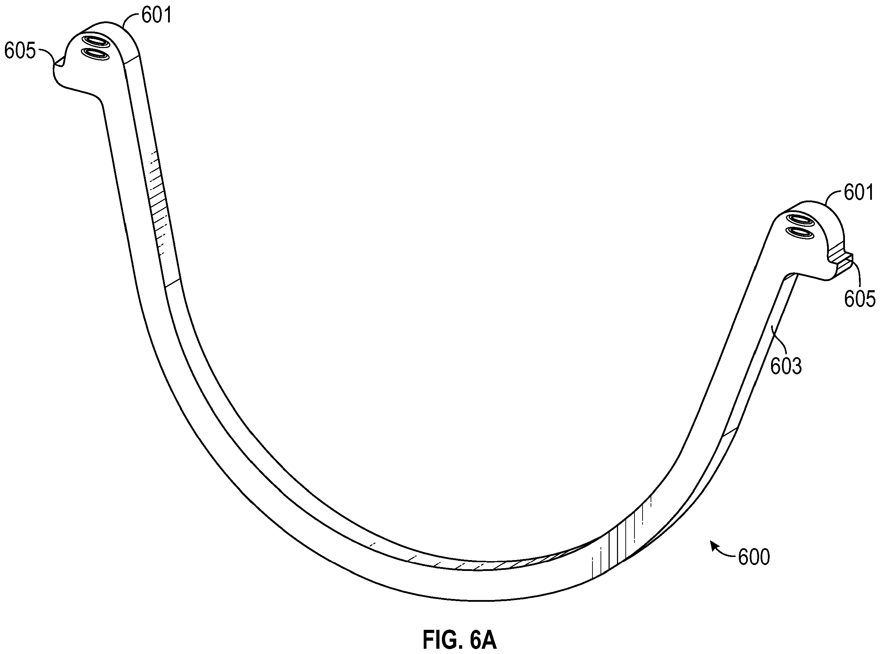

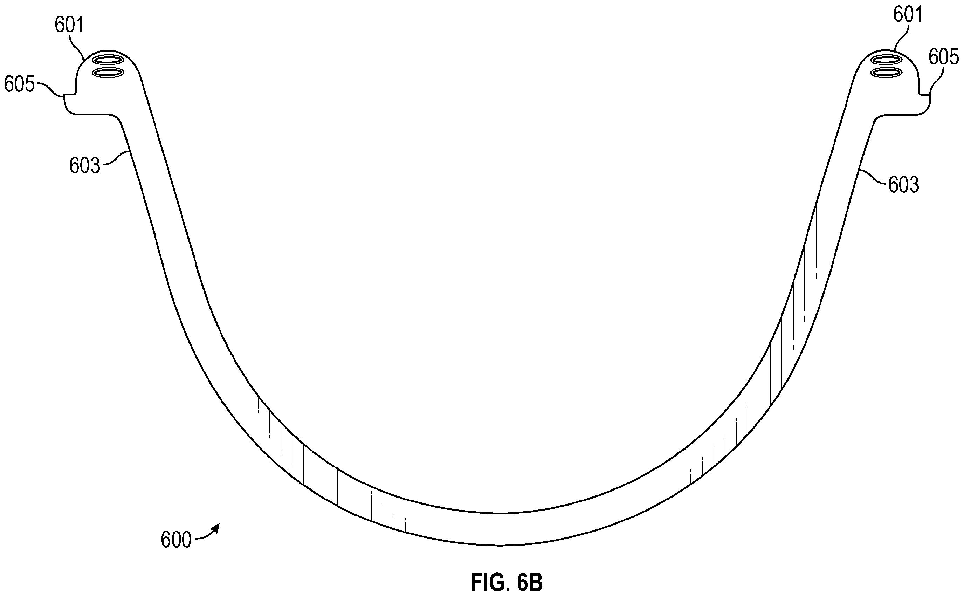

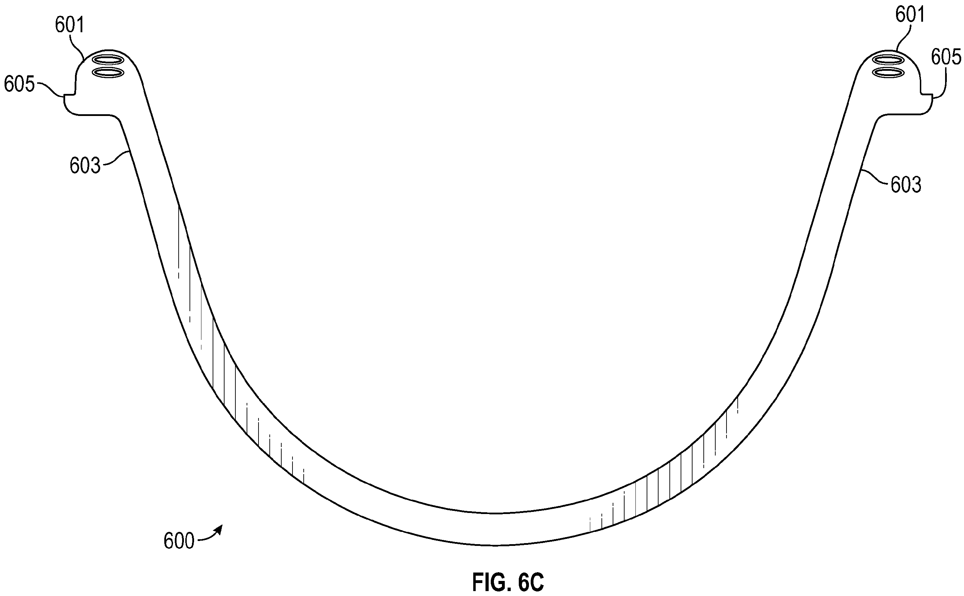

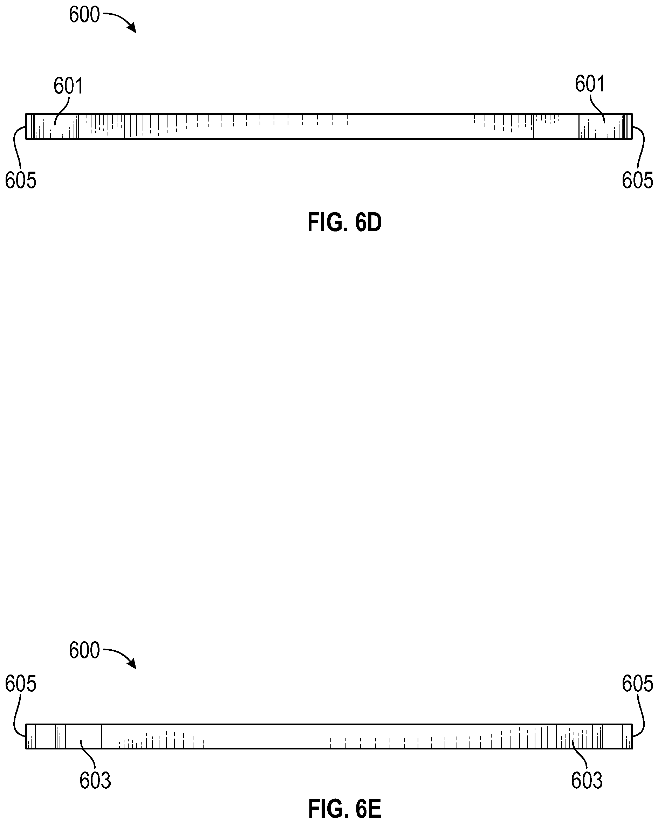

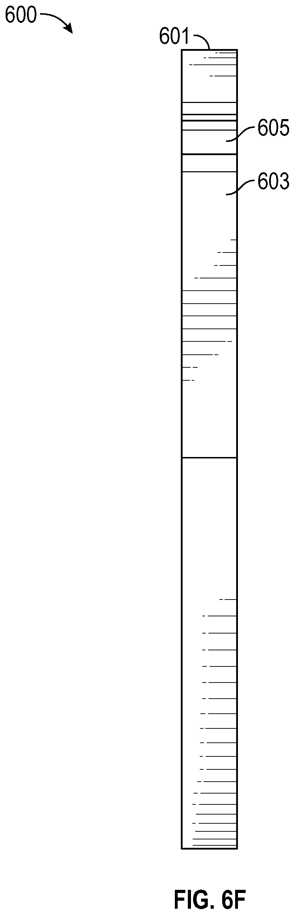

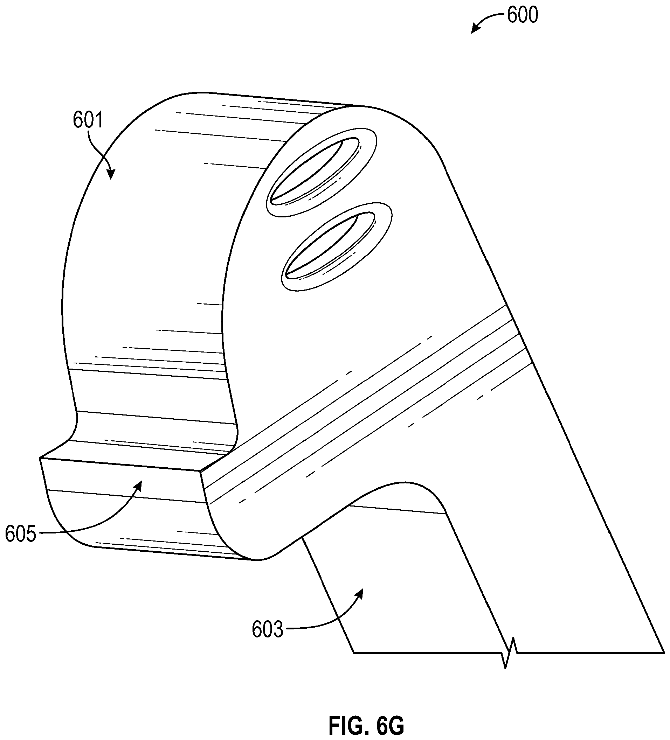

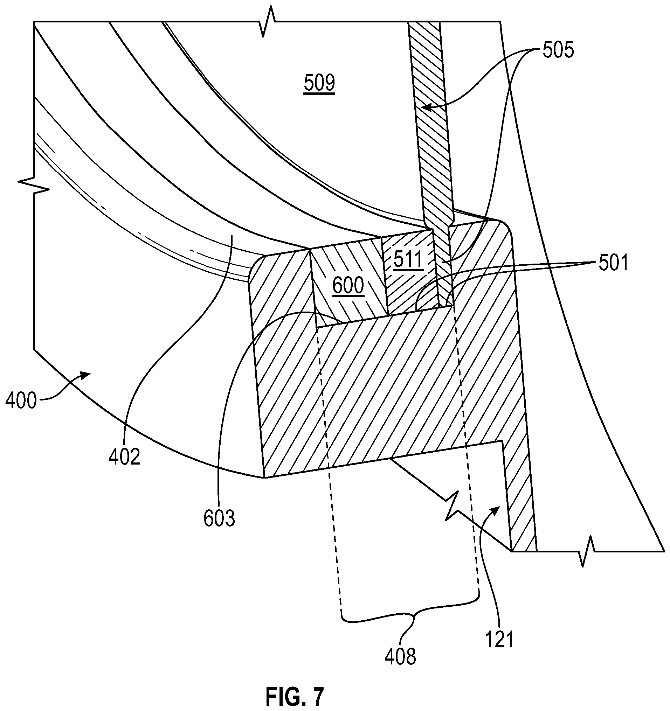

A **FIG. 2** series of figures may comprise **FIG. 2A** through **FIG. 2D****.** These **FIG. 2** series figures may depict vessel neck gasket **500** removably attached to vessel **120.** In contrast, a **FIG. 3** series of figures **(****FIG. 3A** and **FIG. 3B****)** may show vessel neck gasket **500** exploded away from vessel **120,** *i.e.,* vessel neck gasket **500** removably detached from vessel **120;** a **FIG. 4** series may focus on showing neck-gasket-accommodator **400** of vessel **120,** *i.e.,* where vessel neck gasket **500** removably attaches to vessel **120;** a **FIG. 5** series of figures may focus on showing just vessel neck gasket **500;** a **FIG. 6** series may focus on showing just a clamp **600,** which in some embodiments, may help to removably secure vessel neck gasket **500** to neck-gasket-accommodator **400;** **FIG. 7** may show a cross-sectional view of the configuration where vessel neck gasket **500** is removably attached to neck-gasket-accommodator **400.**

Turning back to the **FIG. 2** series of figures, **FIG. 2A** may depict a close up of a front and a top portion of face soaking device **100** showing vessel neck gasket **500** removably attached to vessel **120.** **FIG. 2B** may depict a front view of face soaking device **100** of **FIG. 2A****.** **FIG. 2C** may depict a top view of a front portion of face soaking device **100** of **FIG. 2A****.** **FIG. 2D** may depict a back view of a front portion of face soaking device **100** of **FIG. 2A****.**

Notes: front views may also be known as a vessel neck gasket view, at least when vessel neck gasket **500** may be attached to vessel **120;** a left side view may be with respect to an observer looking upon the front view, wherein the left side view may be with respect to the observer's left, *i.e.,* the left side of face soaking device **100** (or component thereof) when viewed from the front view.

The **FIG. 3** series of figures may comprise **FIG. 3A** and **FIG. 3B****.** **FIG. 3A** may depict a partial exploded view of a front portion of face soaking device **100** of **FIG. 2A****,** shown from a top front perspective view. **FIG. 3B** may depict a partial exploded view of a front portion of face soaking device **100** of **FIG. 2A****,** shown from a top back perspective view. In both **FIG. 3A** and in **FIG. 3B****,** vessel neck gasket **500** may be shown removed away from vessel **120.** In both **FIG. 3A** and in **FIG. 3B****,** portions of neck-gasket-accommodator **400** may be shown as vessel neck gasket **500** may be shown removed away from vessel **120.** In both **FIG. 3A** and in **FIG. 3B****,** clamp **600** may also be shown removed away from vessel **120.**

In some embodiments vessel neck gasket **500** may be attached to neck-gasket-accommodator **400** by use of clamp **600.** *See e.g.,* **FIG. 3A****,** **FIG. 3B****,** and **FIG. 7****.**

The **FIG. 4** series of figures may comprise **FIG. 4A** through **FIG. 4D****.** As noted, these **FIG. 4** series may focus on showing neck-gasket-accommodator **400** of vessel **120,** *i.e.,* where vessel neck gasket **500** removably attaches to vessel **120.** Note, vessel neck gasket **500** may not be shown in the **FIG. 4** series of figures. Note, vessel neck gasket **500** may not be shown in the **FIG. 4** series of figures. Note, clamp **600** may not be shown in the **FIG. 4** series of figures. **FIG. 4A** may depict a close up of a front and a top portion of face soaking device **100** of **FIG. 2A****.** **FIG. 4B** may depict a front view of face soaking device **100** of **FIG. 4A****.** **FIG. 4C** may depict a top view of a front portion of face soaking device **100** of **FIG. 4A****.** **FIG. 4D** may depict a back view of a front portion of face soaking device **100** of **FIG. 4A. FIG. 4A** may correspond to **FIG. 2A****,** **FIG. 4B** may correspond to **FIG. 2B****,** **FIG. 4C** may correspond to **FIG. 2C****,** and **FIG. 4D** may correspond to **FIG. 2D****;** but in these **FIG. 4** series of figures vessel neck gasket **500** and clamp **600** may be removed from vessel **120,** so neck-gasket-accommodator **400** may be visible.

The **FIG. 5** series of figures may comprise **FIG. 5A** through **FIG. 5G****.** As noted, these **FIG. 5** series of figures may focus on showing just vessel neck gasket **500.** **FIG. 5A** may depict an exploded view of vessel neck gasket **500;** wherein a carrier **511** may be separated from a flexible member **505,** shown from a top front perspective view. **FIG. 5B** may depict the assembled vessel neck gasket **500,** shown from a top front perspective view. **FIG. 5C** may depict the assembled vessel neck gasket **500,** shown from a front view. **FIG. 5D** may depict the assembled vessel neck gasket **500,** shown from a back view. **FIG. 5E** may depict the assembled vessel neck gasket **500,** shown from a top view. **FIG. 5F** may depict the assembled vessel neck gasket **500,** shown from a bottom view. **FIG. 5G** may depict the assembled vessel neck gasket **500,** shown from a left side view. (A right side view may be a mirror image of **FIG. 5G****.)**

The **FIG. 6** series of figures may comprise **FIG. 6A** through **FIG. 6G****.** As noted, these **FIG. 6** series of figures may focus on showing just clamp **600.** **FIG. 6A** may depict clamp **600,** shown from a top front perspective view. **FIG. 6B** may depict clamp **600,** shown from a front view. **FIG. 6C** may depict clamp **600,** shown from a back view. **FIG. 6D** may depict clamp **600,** shown from a top view. **FIG. 6E** may depict clamp **600,** shown from a bottom view. **FIG. 6F** may depict clamp **600,** shown from a side view. **FIG. 6G** may depict a close up of one end (a left end) of clamp **600,** shown from a top front perspective view.

**FIG. 7** may depict a cross-sectional view along **sectional-line 7 -7**, that is shown in **FIG. 2B****.** Transverse width cross-sections of neck-gasket-accommodator **400,** clamp **600,** vessel neck gasket **500** may be seen in **FIG. 7****,** all in their assembled configuration wherein clamp **600** may be assisting in the removable attachment of vessel neck gasket **500** to neck-gasket-accommodator **400** of vessel **120.**

In some embodiments, neck-gasket-accommodator **400,** vessel neck gasket **500,** and clamp **600** may all communicate together forming the primary water tight seal. Compare **FIG. 2A** against **FIG. 3A****.** In **FIG. 2A** the primary water tight seal may be intact; whereas, **FIG. 3A** may show how the primary water tight seal may be formed or is unformed.

In some embodiments, components for forming the primary water tight seal may comprise: neck-gasket-accommodator **400,** vessel neck gasket **500,** and clamp **600** (or clamp **800,** clamp **900,** clamp **1000,** or clamp **1100).** In some embodiments, neck-gasket-accommodator **400** may be a structural member located on a side wall *(e.g.,* wall **121)** of vessel **120.** In some embodiments, neck-gasket-accommodator **400** may have a contour **402.** In some embodiments, contour **402** may tracks an open path that may be below a top rim **122** of the vessel **120.** In some embodiments, this open path may be continuous. In some embodiments, extending into the contour **402** may be a receiving-channel **408.** *See e.g.,* **FIG. 4A****.** In some embodiments, vessel neck gasket **500** may comprise a mating edge **501.** *See e.g.,* **FIG. 5A****.** In some embodiments, at least portions of mating edge **501** may fit within receiving-channel **408.** *See e.g.,* **FIG. 3A****,** **FIG. 7****,** **FIG. 8****,** **FIG. 9****,** **FIG. 10****,** and **FIG. 11****.** In some embodiments, the clamp *(e.g.,* **600, 800, 900, 1000,** or **1100)** may also be a structural member. In some embodiments, the clamp *(e.g.,* **600, 800, 900, 1000,** or **1100)** may comprise a mating-wall-edge **603.** *See e.g.,* **FIG. 6A****.** In some embodiments, at least portions of mating-wall-edge **603** may fit within receiving-channel **408.** *See e.g.,* **FIG. 3A****,** **FIG. 7****,** **FIG. 8****,** **FIG. 9****,** **FIG. 10****,** and **FIG. 11****.** In some embodiments, a width of a portion of the clamp *(e.g.,* **600, 800, 900, 1000,** or **1100),** a width of vessel neck gasket **500,** and a width of the receiving-channel **408** may be sized such that when the at least portions of mating-wall-edge **603** and the at least portions of mating edge **501** may be received into receiving-channel **408,** the primary water tight seal is formed between a portion of receiving-channel **408** and some portions of vessel neck gasket **500.** *See e.g.,* **FIG. 3A****,** **FIG. 7****,** **FIG. 8****,** **FIG. 9****,** **FIG. 10****,** and **FIG. 11****.** In some embodiments, clamp **600** (and other disclosed clamps herein) and vessel neck gasket **500** may be removable from receiving-channel **408.** *See e.g.,* **FIG. 3A****.**

In some embodiments, in at least one wall **121** of vessel **120** may be neck-gasket-accommodator **400.** In some embodiments, at least one wall **121** of vessel **120** may comprise neck-gasket-accommodator **400.** Many figures show neck-gasket-accommodator **400** located in a front wall of face soaking device **100.** In some embodiments, neck-gasket-accommodator **400** may be formed in the front wall of vessel **120.** *See e.g.,* the **FIG. 4** series of figures, such as, **FIG. 4A** and **FIG. 4C****.**

In some embodiments, neck-gasket-accommodator **400** may comprise contour **402.** Contour **402** may generally track an overall shape of neck-gasket-accommodator **400.** Contour **402** may comprise one or more surfaces. In some embodiments, such surfaces may face one or more of: each other, face the front of a face soaking device, face the back of a face soaking device, and/or face away from an upper surface of a bottom interior surface of vessel **120.** *See e.g.,* **FIG. 4A** and **FIG. 4B****.**

In some embodiments, neck-gasket-accommodator **400** may comprise contour **402** in at least one wall **121** that runs below rim **122** of vessel **120.** In some embodiments, contour **402** begins where a surface of neck-gasket-accommodator **400** first runs below rim **122** and contour **402** continues until ending where the surface of neck-gasket-accommodator **400** runs back up to rim **122.** In some embodiments, where contour **402** begins and where contour **402** ends may be separated by horizontal width **404.** In some embodiments, contour **338** has maximum vertical length **406** from a height of rim **122** to a lowest point on contour **402.** *See e.g.,* **FIG. 4B****.**

In some embodiments, horizontal width **404** may be greater than or equal to a diameter of a neck of the user. In some embodiments, maximum vertical length **406** may be greater than or equal to half of a diameter of the neck of the user. For example, some large adult men may have a neck circumference of about 21 inches, which results in a neck diameter of about 6.69 inches. For example, and without limiting the scope of the present invention, in some embodiments, horizontal width **404** may be 7 to 11 inches. For example, and without limiting the scope of the present invention, in some embodiments, maximum vertical length **406** may be 3.5 to 7 inches.

Neck-gasket-accommodator **400** may comprise a maximum vertical length **406** greater than a second portion of the neck region of the user extending from rim **122** towards at least one base **125** of vessel **120,** extending to a bottom most portion of neck-gasket-accommodator **400.** *See e.g.,* **FIG. 4B****.** The second portion of the neck region may be a vertical area of a front of the user's neck, *i.e.* the soft tissue side of the neck, where the neck may contact vessel neck gasket **500.** Neck-gasket-accommodator **400** may have a horizontal width **404** greater than a third portion of the neck region of the user centered in a horizontal width (*e.g.*, transverse-width or from right to left) of vessel **120,** extending from rim **122** to opposing rim **122** across an opening that neck-gasket-accommodator **400** creates. *See e.g.,* **FIG. 4B****.** The third portion of the neck region may be a horizontal area of a front of the user's neck, *i.e.* the soft tissue side of the neck, where the neck may contact vessel neck gasket **500.**

Note with respect, to the first portion, the second portion, and the third portion of the neck region of the user, the first portion may comprise the second portion and the third portion. That is, the second portion may define a vertical dimension of the first portion and the third portion may define a horizontal dimension of the first portion. For example, and without limiting the scope of the present invention, this first portion of the neck region may be a portion of the neck what may correspond to where an Adam's Apple of a neck may be located; and including up two inches from that Adam's Apple region or a corresponding region on a neck with no Adam's Apple.

In some embodiments, an overall shape of neck-gasket-accommodator **400,** with respect to at least one base **125** (*see* **FIG. 1** for at least one base **125**) or as viewed from a front of wall **121** that has neck-gasket-accommodator **400,** may be shaped suitable to receive vessel neck gasket **500** and the first portion of the neck region of the user when in use in a given face soaking device embodiment *(e.g.,* face soaking device **100**). *See e.g.,* **FIG. 4B** wherein the overall shape of contour **402** of neck-gasket-accommodator **400** may be: curved; rounded; semicircular; semi-elliptical; U-shaped; horseshoe shaped, semi-oval; an arc of a partial circle; an arc of a partial ellipse; an arc of a partial oval; one third to three thirds of a circle; one third to three thirds of an oval; one third to three thirds of an ellipse; and/or the like; *e.g.,* when viewed from the front of face soaking device **100.**

In some embodiments, a shape *(i.e.,* the overall shape) of contour **402** as viewed from a front of face soaking device **100** may be selected from the group comprising: curved; rounded; semicircular; semi-elliptical; U-shaped; horseshoe shaped, semi-oval; an arc of a partial circle; an arc of a partial ellipse; an arc of a partial oval; one third to three thirds of a circle; one third to three thirds of an oval; one third to three thirds of an ellipse; a shape approximating an open-ended polygon; and/or the like. *See e.g.,* **FIG. 4B****.**

In some embodiments, the open path of contour **402** may be substantially *(i.e.,* not geometrically perfectly) shaped, as viewed from a front of the side wall, as one of the following: curved; rounded; semicircular; semi-elliptical; U-shaped; horseshoe shaped, semi-oval; an arc of a partial circle; an arc of a partial ellipse; an arc of a partial oval; one third to three thirds of a circle; one third to three thirds of an oval; one third to three thirds of an ellipse; a shape approximating an open-ended polygon; and/or the like.

In some embodiments, the open path of contour **402** may be a regular or an irregular polygon that is not closed *(i.e.,* an open shape) or semi-polygon suitable to that is not closed (*i.e.,* an open shape) suitable to receive bottom portions of vessel neck gasket **500** (*e.g.,* mating edge **501**) and the first portion of the neck region of the user when in use. This embodiment is not shown in the figures; however, it should be obvious to one of ordinary skill in the art that the overall shape semi-circular neck-gasket-accommodator **400** shown **FIG. 4B** may be modified into the open regular or the open irregular polygon or the open semi-polygon of contour **402** and that such a contour may continue to be within the scope of this invention.

Note, in some embodiments, neck-gasket-accommodator **400** may be termed a "gasket-accommodator."

In some embodiments, contour **402** may comprise at least one surface facing away from an upper surface of a bottom interior surface of vessel **120.** In some embodiments, along a length of contour **402** may be a receiving-channel **408.** *See e.g.,* **FIG. 4A** and **FIG. 7** for a cross-sectional view of this receiving-channel **408.** Receiving-channel **408** may be channel or groove cut into contour **402,** substantially along a length of contour **402.** *See e.g.,* **FIG. 4A****.** A width of receiving-channel **408** may be sized to frictionally fit widths of one or more of: vessel neck gasket **500** *(e.g.,* flexible member **505** plus carrier **511**), and/or clamp **600;** such that the primary water tight seal is formed. *See e.g.,* **FIG. 7****.**

In some embodiments, a cross-section of the receiving-channel **408** may be substantially *(i.e.,* not geometrically perfectly) shaped as one of the following: curved; rounded; semi-circular; semi-elliptical; U-shaped; horseshoe shaped, semi-oval; an arc of a partial circle; an arc of a partial ellipse; an arc of a partial oval; one third to three thirds of a circle; one third to three thirds of an oval; one third to three thirds of an ellipse; or a shape approximating an open-ended polygon. *See e.g.,* **FIG. 7****,** **FIG. 8****,** **FIG. 9****,** **FIG. 10****,** and **FIG. 11****.**

In some embodiments, a direction that receiving-channel **408** extends into contour **402** may be substantially parallel with a major plane of side wall **121.** *See e.g.,* **FIG. 7****,** **FIG. 8****,** **FIG. 9****,** **FIG. 10****,** **FIG. 11****,** and **FIG. 12E****.** In some embodiments, the direction that receiving-channel **408** extends into contour **402** may not be perpendicular (nor substantially perpendicular) with the major plane of side wall **121.** The major plane of side wall **121** may be substantially collinear with an external surface of side wall **121.**

In some embodiments, bottom portions of vessel neck gasket **500** may be configured to fit to *(e.g.,* mate to) contour **402** of neck-gasket-accommodator **400** in at least one wall **121** of vessel **120.** In some embodiments, bottom portions of vessel neck gasket **500** may be configured to fit to *(e.g.,* mate to) receiving-channel **408** in contour **402** of neck-gasket-accommodator **400** in at least one wall **121** of vessel **120.** *See e.g.,* **FIG. 3A****,** **FIG. 4A****,** **FIG. 5A****,** and **FIG. 7****.** Surface area of mating edge **501** of vessel neck gasket **500** may be equal to, greater than, or less than, as compared against surface area of neck-gasket-accommodator **400.** Vessel neck gasket **500** may comprise a mating edge **501** complimentary to at least some surfaces of contour **402** of neck-gasket-accommodator **400** in at least one wall **121** of vessel **120.** Vessel neck gasket **500** may comprise a top edge **503.** In some embodiments, top edge **503** and mating edge **501** together may define a perimeter of vessel neck gasket **500.** In some embodiments, top edge **503** and mating edge **501** may together circumscribe and define a closed shape for vessel neck gasket **500.** In some embodiments, top edge **503** and mating edge **501** may each curve for at least some portion of the given edge. In some embodiments, a radius of curvature for top edge **503** may be different for a radius of curvature for edge **501.** *See e.g.,* **FIG. 5A****.** In some embodiments, top edge **503** may be accommodative to receiving the first portion of the neck region of the user. In some embodiments, when vessel neck gasket **500** may be attached to neck-gasket-accommodator **400,** *e.g.,* as in the **FIG. 2** series of figures (*e.g.,* **FIG. 2A**), some portions of top edge **503** may extend above rim **122** (*e.g.,* **FIG. 2B**). In some embodiments, mating edge **501** of vessel neck gasket **500** may be attached to contour **402** of neck-gasket-accommodator **400** by a vessel neck gasket attachment means. In some embodiments, mating edge **501** of vessel neck gasket **500** may be received into receiving-channel **408** of contour **402.**

In some embodiments, the vessel neck gasket attachment means may comprise clamp **600.** Or in some embodiments, mating edge **501** of vessel neck gasket **500** may be attached to either an exterior wall surface or an interior wall surface of at least one wall **121,** at a fixed distance from contour **402** of neck-gasket-accommodator **400** by the vessel neck gasket attachment means. For example, and without limiting the scope of the present invention, in some embodiments, the fixed distance may be one inch or less. In other embodiments, the fixed distance may be other distances.

In some embodiments, vessel neck gasket **500** may be removable from vessel **120.** In some embodiments, vessel neck gasket **500** may be removable from neck-gasket-accommodator **400.** Such embodiments may facilitate switching out vessel neck gasket **500** in the event of wear and tear and/or damage to an installed vessel neck gasket **500.** In some other embodiments, vessel neck gasket **500** may not be removed from vessel **120** once installed.

In some embodiments, vessel neck gasket **500** may be a subassembly comprising two parts: a flexible member **505** and a carrier **511.** In some embodiments, vessel neck gasket **500** may comprise flexible member **505** and carrier **511.** In some embodiments, flexible member **505** and carrier **511** may be attached to each other. *See e.g.,* **FIG. 5A****.**

In some embodiments, flexible member **505** may be substantially planar with an internal surface **507** *(see* **FIG. 5D**) and an external surface **509** (*see* **FIG. 5A**) disposed opposite of internal surface **507.** Portions of either external surface **509** or internal surface **507** may form the secondary water tight seal with the neck region, when the user rests their neck against portions of vessel neck gasket **500.** In some embodiments, a portion of internal surface **507** may physically contact some of the liquid when the liquid may be held within the internal volume of vessel **120.**

In some embodiments, flexible member **505** may be a flexible sheet. In some embodiments, this flexible sheet may be shaped generally with mating edge **501** that is complimentary to cover portions (*e.g*., receiving-channel **408**) of neck-gasket-accommodator **400** of vessel **120.** In some embodiments, this flexible sheet may be shaped generally with mating edge **501** that is complimentary to cover portions of receiving-channel **408.** In some embodiments, this flexible sheet may comprise a gasket along the bottom of the sheet. In some embodiments, this gasket may be mating edge **501.** *See e.g.,* **FIG. 5A** and **FIG. 5F****.**

In some embodiments, flexible member **505** may be substantially constructed of one or more materials (*i.e*., materials of construction) suitable for forming water tight seals against human skin (or against an exterior portion of a terrestrial vertebrates body) and/or against the vessel (*e.g*. vessel **120**), and/or suitable for being comfortable when touching human skin. In some embodiments, flexible member **505** may be constructed of one or more of elastomers comprising silicone, rubber, neoprene, nitrile, vinyl, polyethylene, polypropylene, and/or any other material suitable for forming water tight seals against human skin and/or against the vessel (*e.g.* vessel **120**), and/or suitable for being comfortable when touching human skin. In some embodiments, the rubber may be natural rubber. In some embodiments, the rubber may be synthetic, including latex free. In some embodiments, flexible member **505** may be compressible. For example, and without limiting the scope of the present invention, when vessel neck gasket **500** may be attached to neck-gasket-accommodator **400,** portions of internal surface **507** and external surface **509** that may be residing within receiving-channel **408** of neck-gasket-accommodator **400,** may be compressed; *see e*.*g*., **FIG. 7**. Such compression of portions of flexible member **505** within receiving-channel **408** may aid in forming the primary water tight seal. Compare, **FIG. 5F** and **FIG. 5G** may show flexible member **505** in a non-compressed state. In some embodiments, portions of flexible member **505** may be compressed by 5% to 95% as compared to non-compressed states of flexible member **505.**

In some embodiments, a shape *(e.g.,* an overall shape) of receiving-channel **408** as viewed in a cross-section may be selected from the group comprising: one third to three thirds of a circle; one third to three thirds of an oval; one third to three thirds of an ellipse; a "U" shape, a horseshoe shape; a regular polygon open at a top (with or without rounded corners), an irregular polygon open at a top (with or without rounded corners); a semi-polygon open at a top (with or without rounded corners); and/or the like; with an arc of the partial circle, partial oval, partial ellipse, or the horseshoe shape oriented downwards towards at least one base **125.** *See e.g.,* **FIG. 7****,** **FIG. 8****,** **FIG. 9****,** **FIG. 10****, and** **FIG. 11****.**

In some embodiments, internal surface **507** and external surface **509** may be constructed of different elastomers. That is, flexible member **505** may be a laminate or comprised of planar layers. Internal surface **507** may be constructed of an elastomer with a focus on water impermeability. External surface **509** may be constructed of an elastomer with a focus on comfort to the user, *i.e.,* an elastomer with a soft outer surface and/or a non-tacky outer surface. Such two different elastomers may be joined into a single flexible composite member of flexible member **505.** The means for joining internal surface **507** to external surface **509** may be by solvent bonding, heat welding, ultrasonic welding, chemical adhesive/sealant, mechanical fasteners (*e.g*., stitching and/or staples, or the like), and/or the like.

In some embodiments, flexible member **505** may be manufactured by die cutting, stamping, molding, and/or 3D printing.

In some embodiments, carrier **511** may be attached to flexible member **505.** The means for joining carrier **511** to flexible member **505** may be by solvent bonding, heat welding, ultrasonic welding, chemical adhesive/sealant, mechanical fasteners (*e.g*., stitching and/or staples, or the like), and/or the like. *See e.g.,* **FIG. 5A** and **FIG. 5B****.** In some embodiments, carrier **511** may be integral with flexible member **505.** In some embodiments, carrier **511** may be a structural member. In some embodiments, carrier **511** may be rigid to semi-rigid. In some embodiments, carrier **511** may be substantially constructed from one or more thermoformed plastics, metal, wood, composite, laminate, and/or the like. In some embodiments, carrier **511** may have a higher durometer as compared against a durometer of flexible member **505.** In some embodiments, carrier **511** may impart structural rigidity to bottom portions of flexible member **505,** which may facilitate loading or feeding of bottom portions of vessel neck gasket **500** into neck-gasket-accommodator **400** receiving-channel **408.** *See e.g.,* **FIG. 3A** and **FIG. 3B****.** In some embodiments, carrier **511** may have a shape that is complimentary to bottom surfaces of flexible member **505,** such as mating edge **501.** In some embodiments, both carrier **511** and flexible member **505** may share mating edge **501.** *See e.g.,* **FIG. 3A****,** **FIG. 5A****,** **FIG. 5B****,** **FIG. 7****,** **FIG. 8****,** **FIG. 9****,** **FIG. 10****,** and **FIG. 11****.**

In some embodiments, carrier **511** may be shaped complimentary to a shape of the open path of contour **402.** In some embodiments, mating edge **501** may be shaped complimentary to a shape of receiving-channel **408.** *See e.g.,* **FIG. 3A****,** **FIG. 7****,** **FIG. 8****,** **FIG. 9****,** **FIG. 10****,** and **FIG. 11****.**

In some embodiments, carrier **511** may be manufactured by die cutting, stamping, molding, extrusion, and/or 3D printing.

In some embodiments, the vessel neck gasket attachment means may be selected from one or more of: a friction fit, heat welding, ultrasonic welding, solvent bonding, chemical adhesives and/or sealants, mechanical fasteners, and/or the like. Use of clamp **600** may an example of a frictional fit and/or a type of mechanical fastener. Recall, the vessel neck gasket attachment means may be how vessel neck gasket **500** may be attached to neck-gasket-accommodator **400.**

In some embodiments, an embodiment of face soaking device **100** may comprise at least these elements: vessel neck gasket **500,** clamp **600,** and vessel **120** with neck-gasket-accommodator **400** of at least one wall **121** of vessel **120.** In some embodiments, a vessel neck gasket subassembly may comprise at least these parts: vessel neck gasket **500,** clamp **600,** and neck-gasket-accommodator **400.** Neck-gasket-accommodator **400** may be a portion or region of a given vessel embodiment, such as vessel **120** embodiments.

In some embodiments vessel neck gasket **500** may be attached to neck-gasket-accommodator **400** by use of clamp **600.** *See e.g.,* **FIG. 3A****,** **FIG. 3B****,** and **FIG. 7****.** In some embodiments such an attachment may form the primary water tight seal. In some embodiments clamp **600** may be shaped to complimentary fit to neck-gasket-accommodator **400,** with a portion of vessel neck gasket **500** sandwiched between clamp **240** and contour **402** of neck-gasket-accommodator **400,** forming the primary water tight seal. *See e.g.,* **FIG. 3A****,** **FIG. 3B****,** and **FIG. 7**. In some embodiments, contour **402** may comprise at least one surface facing away from an upper surface of a bottom interior surface of vessel **120.** The Bottom interior surface may be an interior surface of vessel **120.** A width of receiving-channel **408** (of neck-gasket-accommodator **400**) may be sized to frictionally fit widths of one or more of: vessel neck gasket **500** *(i.e.,* flexible member **505** plus carrier **511**), and/or clamp **600;** such that the primary water tight seal is formed. *See e.g.,* **FIG. 7****.**

In some embodiments, clamp **600** fit attachment to contour **402** of neck-gasket-accommodator **400** may be removable. In some embodiments, at least some portions of clamp 600 may fit into portions of receiving-channel **408.** In some embodiments, clamp **600** fit attachment to contour **402** of neck-gasket-accommodator **400** may be a frictional fit. In some embodiments, clamp **600** fit attachment to contour **402** of neck-gasket-accommodator **400** may be from one or more snap fits.

In some embodiments, clamp **600** may be constructed (or substantially) of a semi-rigid to rigid material of construction. For example, and without limiting the scope of the present invention, clamp **600** may be substantially constructed of one or more of a thermoformed plastic, metal, wood, composite, laminate, and/or the like. In some embodiments, clamp **600** and carrier **511** may be substantially constructed of the same types of materials. In some embodiments, clamp **600** may be manufactured via die cutting, stamping, molding (*e.g*., injection molding), extrusion, and/or 3D printing. In some embodiments, at least some of the above characteristics (*e.g*., rigidity and/or materials of construction) of clamp **600** may be shared with clamps **800, 900, 1000,** and **1100.** However, as show, clamps **800, 900, 1000,** and **1100** may have different geometry and/or structures from clamp **600.**

A **FIG. 6A** and **FIG. 6G** may depict snap latch structure illustrating how clamp **600** (of the **FIG. 3** series) may removably couple with neck-gasket-accommodator **400.** Such snap latch connections may work to assist in removably coupling clamp **600** to neck-gasket-accommodator **400.**

In some embodiments, neck-gasket-accommodator **400** may comprises two opposing opening wall-edges **410.** In some embodiments, the two opposing opening wall-edges **410** may define where neck-gasket-accommodator **400** begins. *See e.g.,* **FIG. 4A** and **FIG. 4C****.** In some embodiments, each opening wall-edge **410** *(e.g.,* left and right) may be separated from the other by at least the at least horizontal width **404** of neck-gasket-accommodator **400** *(see e.g.,* **FIG. 4B** for horizontal width **404**). In some embodiments, each opening wall-edge **410** may descend downwards in a direction towards at least one base **125** from rim **122.** In some embodiments, each opening wall-edge **410** may be substantially flat *(i.e.,* a substantially flat surface). *See e.g.,* **FIG. 4A** and **FIG. 4C****.**

In some embodiments, clamp **600** may comprise two terminal ends **601** disposed opposite of each other. *See e.g.,* **FIG. 6A****,** **FIG. 6B****,** and **FIG. 6C****.** In some embodiments, each terminal end **601** may comprise a mating-wall-edge **603.** In some embodiments, clamp **600** may comprise mating-wall-edges **603.** In some embodiments, at least some portion of a given mating-wall-edge **603** may be located below and/or proximate (*e.g*., within three inches in some embodiments) to a given terminal end **601.** *See e.g.,* **FIG. 6A** which may depict one such mating-wall-edge **603;** *e.g.,* a right mating-wall-edge **603** from the perspective of the user looking at the front of face soaking device **100;** wherein a right mating-wall-edge **603** may be a mirror image of the left mating-wall-edge **603.**

In some embodiments, each mating-wall-edge **603** may be paired with a respective opening wall-edge **410,** such that mating-wall-edge **603** paired to a given opening wall-edge **410** may be complimentary to each other, when clamp **600** may be removably coupled to neck-gasket-accommodator **400.** *See e.g.,* **FIG. 3A** and **FIG. 3B****.** In some embodiments, each mating-wall-edge **603** may be paired with a respective opening wall-edge **410,** such that mating-wall-edge **603** paired to the respective opening wall-edge **410** may be substantially parallel to each other, when clamp **600** may be removably coupled to neck-gasket-accommodator **400.** For example, and without limiting the scope of the present invention, left opening wall-edge **410** may be paired with left mating-wall-edge **603.** For example, and without limiting the scope of the present invention, right opening wall-edge **410** may be paired with right mating-wall-edge **603.** In some embodiments, each mating-wall-edge **603** may be substantially flat to compliment a substantially flat region on corresponding (paired) opening wall-edge **410.** *See e.g.,* **FIG. 3A** and **FIG. 3B****.**

In some embodiments, when left opening wall-edge **410** may be paired with left mating-wall-edge **603** and right opening wall-edge **410** may be paired with right mating-wall-edge **603** each respective pairing may be a friction fit between the paired and complimentary surfaces.

In some embodiments, each mating-wall-edge **603** may terminate in a snap latch **605.** *See e.g.,* **FIG. 6A****,** **FIG. 6B****,** and **FIG. 6G****.** In some embodiments, each opening wall-edge **410** may comprise a pocket **412,** which may be an indenture extending into the given opening wall-edge **410.** Each such pocket **412** may be sized to removably fit a respective snap latch **605.** In some embodiments, each snap latch **605** may be paired with a respective pocket **412** such that the paired snap latch **605** may removably snap into paired pocket **412** to form a removable snap latch connection at each of the two respective pairings. *See e.g.,* **FIG. 3A** and **FIG. 3B****.** In some embodiments, a paired snap latch **605** may comprise a protrusion that may be removably engage the paired pocket **412.** *See e.g.,* **FIG. 3A** and **FIG. 3B****.**

In some embodiments, release of each removable snap latch connection (left and right) may be accomplished by the user squeezing each terminal end **601** towards each other, which may disengage a given paired snap latch **605** from the paired pocket **412.** In some embodiments, release of clamp **600** from neck-gasket-accommodator **400** may also require the user to pull clamp **600** upwards or away from neck-gasket-accommodator **400.** In some embodiments, there may be structure located proximate (*e.g*., within three inches) to each terminal end **601;** wherein such structure may aid or may facilitate removal *(i.e.,* release) of clamp **600** from neck-gasket-accommodator **400.** For example, and without limiting the scope of the present invention, in some embodiments, this structure may be indentures, such as, divots, to removably receive a tool for pushing the two terminal ends **601** towards each other. *See e.g.,* **FIG. 6A****,** **FIG. 6B****,** **FIG. 6C****,** **FIG. 6G****,** **FIG. 2A****,** **FIG. 2B****,** **FIG. 2D****,** **FIG. 3A****,** and **FIG. 3B****.** For example, and without limiting the scope of the present invention, in some embodiments, this structure may be removably engage pressing fingers from the user for pushing (*e.g*., squeezing) the two terminal ends **601** towards each other.

Some embodiments may be characterized as a "flexible detachable vessel cover" that may comprise: (1) vessel **120** having a top opening and capable of holding the liquid; wherein vessel **120** has neck-gasket-accommodator **400;** and (2) a flexible sheet (*e.g.,* vessel neck gasket **500);** wherein the flexible sheet that may be shaped generally to cover gasket-accommodator **400** of vessel **120;** wherein the flexible sheet has a gasket (*e.g*., mating edge **501)** along the bottom of the flexible sheet; and wherein the bottom of the gasket and a top of gasket-accommodator **400** of vessel **120** are arranged to mate tightly with one another to form the primary water tight seal.

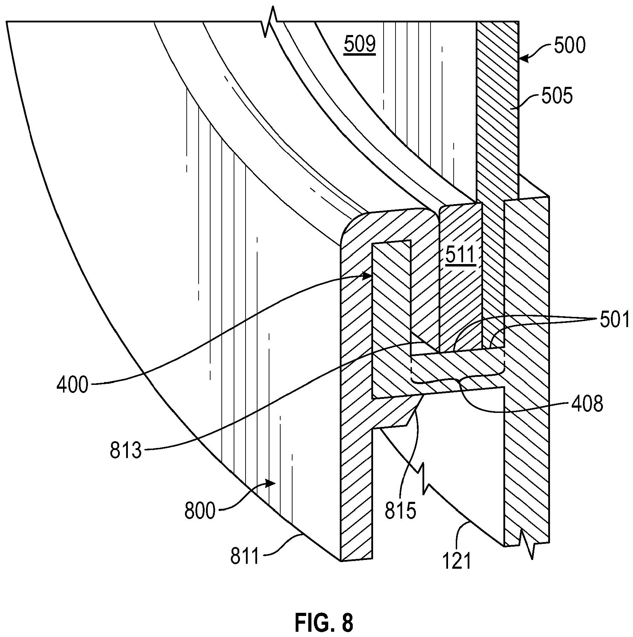

**FIG. 8****,** **FIG. 9****,** **FIG. 10****,** and **FIG. 11** may depict views and embodiments comparable against those shown in **FIG. 7****.** **FIG. 8****,** **FIG. 9****,** **FIG. 10****,** and **FIG. 11** may depict additional embodiments of the clamp, *i.e.,* of clamp **800,** clamp **900,** clamp **1000,** and clamp **1100,** respectively, wherein such clamps may frictionally and removably snap onto an exterior vertical portion of neck-gasket-accommodator **400.** *See e.g.,* **FIG. 8****,** **FIG. 9****,** **FIG. 10****,** and **FIG. 11****.** Compare the clamps of **FIG. 8****,** **FIG. 9****,** **FIG. 10****,** and **FIG. 11** against clamp **600** of **FIG. 7****.**

In some embodiments of face soaking device **100,** the clamp may be selected from: clamp **600,** clamp **800,** clamp **900,** clamp **1000,** clamp **1100,** or the like.

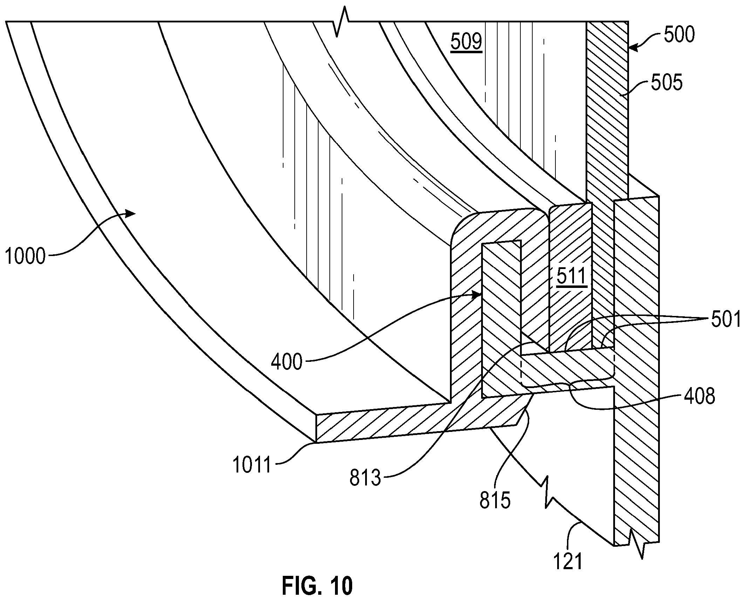

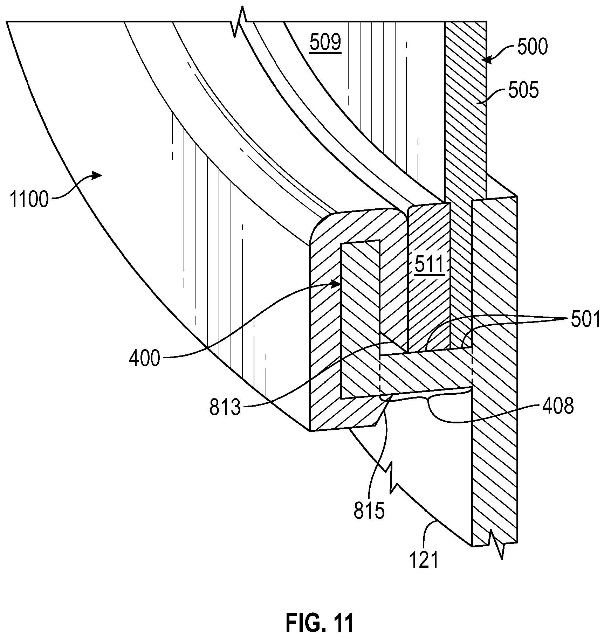

In **FIG. 8****,** clamp **800** may removably and frictionally snap around the exterior vertical portion of neck-gasket-accommodator **400.** In some embodiments, to assist with this removable and frictional snap lock, clamp **800** may comprise a relatively short protrusion, tab **815,** which may protrude substantially perpendicularly from a vertical direction of clamp **800,** such that tab **815** may removably engage a bottom and outward edge of neck-gasket-accommodator **400.** In some embodiments, tab **815** may extend from the given clamp *(e.g.,* clamp **800)** in a direction that may be substantially perpendicular from the major plane of side wall **121.** In some embodiments, tab **815** may snap onto a corner of the neck-gasket-accommodator **400.** In some embodiments, when a given clamp (*e.g*., clamp **800)** may be attached to neck-gasket-accommodator **400,** tab **815** may protrude towards vessel **120.** *See e.g.,* **FIG. 8****.** Tab **815** may have a length that is less than the vertical lengths of clamp **800.** Tab **815** may be an integral structure of clamp **800.** In some embodiments, clamp **1000** and clamp **1100** may also comprise such a tab **815.** *See e.g.,* **FIG. 10** and **FIG. 11****.**

Continuing discussing **FIG. 8****,** in some embodiments, clamp **800** may also comprise finger-pull **811.** Finger-pull **811** may be a vertically oriented finger pull, allowing the user to lift under finger-pull **811** when removing clamp **800** from neck-gasket-accommodator **400.** In some embodiments, finger-pull **811** may be a vertical extension of clamp **800,** extending below and beyond tab **815,** and below where neck-gasket-accommodator **400** ends. Finger-pull **811** may be an integral structure of clamp **800.** In cross-section as shown in **FIG. 8****,** clamp **800** may resemble a stylized letter "f"; wherein the terminal of the ascender curves around to point towards receiving-channel **408;** the stem descends into finger-pull **811;** and there may only one arm being that of tab **815.**

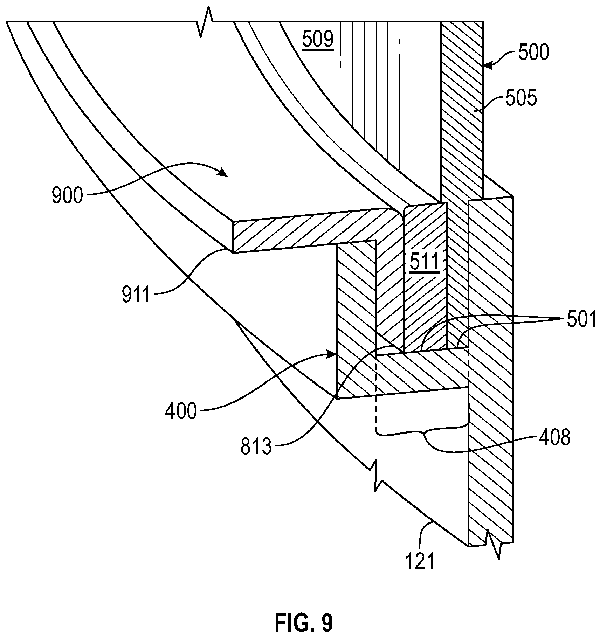

Clamp **900** (shown in **FIG. 9**) and clamp **1000** (shown in **FIG. 10**) may each also comprise similar finger pulls, finger-pull **911** and finger-pull **1011,** respectively. Each of finger-pull **911** and finger-pull **1011,** may extend substantially perpendicularly away from wall 121 shown in **FIG. 9** and in **FIG. 10****,** respectively. *See e.g.,* **FIG. 9** and **FIG. 10****.** A cross-section of clamp **900** may be "L" shaped, where one leg of the "L" is for removable insertion into receiving-channel **408** and the other leg of the "L" is finger-pull **911.** *See* **FIG. 9****.** A cross-section of clamp **1000** may resemble an upside down letter "J." With clamp **1000,** finger-pull **1011** and tab **815** may be collinear but extending in opposite directions of each other, with finger-pull **1011** extending away from vessel **120** and with tab **815** extending towards vessel **120.** *See* **FIG. 10****.**

Although all three finger pulls (*e.g*., finger-pull **811,** finger-pull **911,** and, finger-pull **1011)** may be structurally different, each may serve a similar or same purpose, in aiding the user in removing the given clamp from neck-gasket-accommodator **400,** by providing explicit structure to be engaged by fingers for pulling upwards and away from vessel **120.**

Additionally, clamp **800** may comprise chamfer **813,** which may be located a terminal portion of clamp **800** that is removably retained in receiving-channel **408.** Chamfer **813** may be a chamfer and may aid in inserting clamp **800** into receiving-channel **408** and against portions of vessel neck gasket **500** that also may removably reside within receiving-channel **408.** Clamp **600,** clamp **900,** clamp **1000,** and clamp **1100** may also comprise such a chamfer **813.** Chamfer **813** may also aid use of a given finger pull *(e.g.,* finger-pull **811,** finger-pull **911,** and, finger-pull **1011),** by providing a small region of void space within receiving-channel **408,** such that when the user pulls on the given finger pull, the terminal end of the clamp within receiving-channel **408** may articulate *(i.e.,* pivot) into this void region of the given receiving-channel **408.**

Note, with respect to various clamps disclosed herein, such as, but not limited to, clamp **600,** clamp **800,** clamp **900,** clamp **1000,** and clamp **1100,** these clamps may function technically as a wedge, when portions of such clamps may be removably inserted in receiving-channel **408,** creating opposing lateral compression forces against portions of vessel neck gasket **500** *(e.g.,* carrier **511** and flexible member **505)** within receiving-channel **408,** as well as against wall(s) of receiving-channel **408,** which may be surfaces of contour **402.** These lateral compression forces may compress portions of flexible member **505** as noted herein, which may aid in creating the primary water tight seal. *See e.g.,* **FIG. 7****,** **FIG. 8****,** **FIG. 9****,** **FIG. 10****,** and **FIG. 11****.** Additionally, portions of these clamps (*e.g*., clamp **600,** clamp **800,** clamp **900,** clamp **1000,** and clamp **1100),** *e.g*., mating-wall-edge **603,** which may be structure present on each of these clamps, may press against portions of receiving-channel **408,** such as opposing opening wall-edge **410;** and in this functional capacity these clamps may be acting as a clamp (as in a device that presses together so as to hold firmly) and/or as a wedge. That is, when any of these clamps may be inserted into receiving-channel **408,** receiving-channel **408** and the portions of the clamp may be pressing against each other.

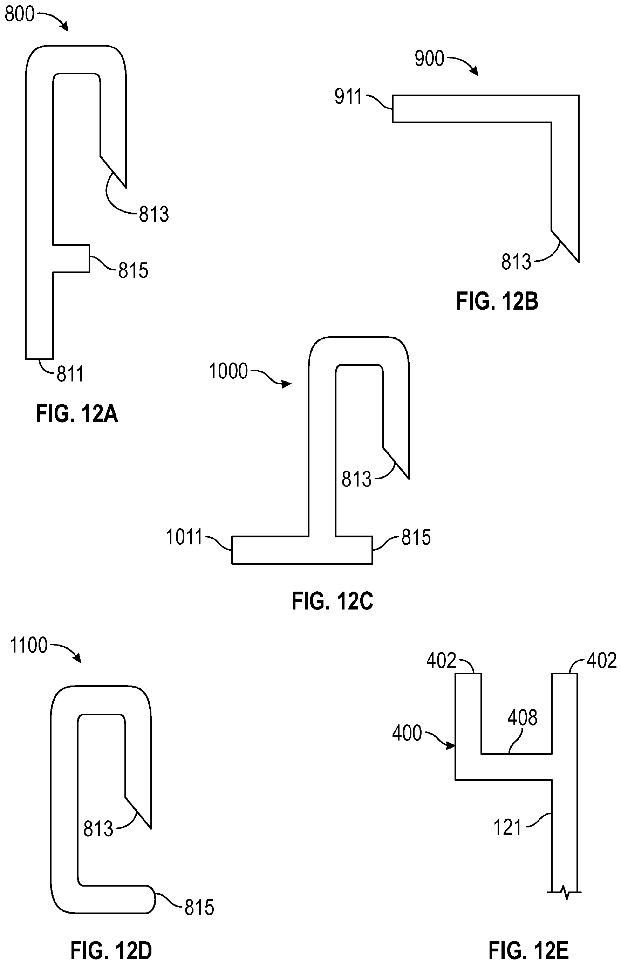

**FIG. 12A** through **FIG. 12D** may depict cross-sectional views of various clamps, such as clamp **800** in **FIG. 12A****;** clamp **900** in **FIG. 12B****;** clamp **1000** in **FIG. 12C****;** and clamp **1100** in **FIG. 12D. FIG. 12E** may depict a cross-sectional view of neck-gasket-accommodator **400;** which may comprise receiving-channel **408** that may receive portions (*e.g*., portions with chamfer **813**) of the various clamps. **FIG. 12A** may show that clamp **800** may resemble a letter "f" in cross-section. **FIG. 12B** may show that clamp **900** may resemble a letter "L" in cross-section. **FIG. 12C** may show that clamp **1000** may resemble a letter "J" in cross-section. **FIG. 12D** may show that clamp **1100** may resemble a letter "C" in cross-section.

In some embodiments, a region comprising neck-gasket-accommodator **400** and comprising where neck-gasket-accommodator joins the side wall **121;** wherein this region, in cross-section, resembles an Arabic number "4." *See e.g.,* **FIG. 12E****,** **FIG. 7****,** **FIG. 8****,** **FIG. 9****,** **FIG. 10****,** and **FIG. 11****.**

In some embodiments, some face soaking device **100** component parts may be substantially constructed of one or more thermoplastics suitable for injection molding and/or 3D printing. For example, and without limiting the scope of the present invention, some face soaking device **100** component parts may be substantially constructed of one or more materials of acrylonitrile-butadiene styrene (ABS), polyvinyl chloride (PVC), polycarbonate, nylon, polypropylene, polyethylene (*e.g*., HDPE), elastomers, rubbers, silicones, fiberglass, and/or the like.

Note with respect to the materials of construction, it is not desired nor intended to thereby unnecessarily limit the present invention by reason of such disclosure.

Note, in some embodiments, such component parts as flexible member **505,** carrier **511,** and/or the various clamps (*e.g*., clamp **600** and clamp **900**) may be manufactured with reliable and quality manufacturing methods of die cutting (stamping) which may be considerably less expensive as compared against creating the tooling for molds of such parts. And other clamp embodiments (*e.g*., clamp **600,** clamp **800,** clamp **900,** clamp **1000,** and clamp **1100**), may be manufacturing by custom extrusion with cuts at appropriate lengths, which may be a reliable and quality manufacturing method that is less expensive than creating the tooling for molds of such parts.

Face soaking devices have been described. The foregoing description of the various exemplary embodiments of the invention has been presented for the purposes of illustration and disclosure. It is not intended to be exhaustive or to limit the invention to the precise form disclosed. Many modifications and variations are possible in light of the above teaching.

While the invention has been described in connection with what is presently considered to be the most practical and preferred embodiments, it is to be understood that the invention is not to be limited to the disclosed embodiments, but on the contrary, is intended to cover various modifications and equivalent arrangements included within the scope of the appended claims.

## Claims

1. A face soaking device (100) comprising:
a vessel (120); and
components for forming a primary water tight seal comprising:
a neck-gasket-accommodator (400) for accommodating a vessel neck gasket (500) that is configured to comfortably accommodate a portion of a user's neck, wherein the neck gasket accommodator (400) is a first structural member located on a side wall (121) of the vessel (120); wherein the neck-gasket-accommodator (400) has a contour (402) that tracks an open path that is below a top rim (122) of the vessel (120); wherein extending into the contour (402) is a receiving-channel (408);
the vessel neck gasket (500), comprising a mating edge (501); wherein at least portions of the mating edge (501) fit within the receiving-channel (408); and
a clamp (600) that is a second structural member with a mating-wall-edge (603); wherein at least portions of the mating-wall-edge (603) fit within the receiving-channel (408);
wherein a width of a portion of the clamp (600), a width of the vessel neck gasket (500), and a width of the receiving-channel (408) are sized such that when the at least portions of the mating-wall-edge (603) and the at least portions of the mating edge (501) are received into the receiving-channel (408), the primary water tight seal is formed between a portion of the receiving-channel (408) and some portions of the vessel neck gasket (500), wherein a portion of the vessel neck gasket (500) is sandwiched between the clamp (600) and the contour (402) of the neck-gasket-accommodator (400).

2. The face soaking device (100) according to claim 1, wherein the open path of the contour (402) is continuous.

3. The face soaking device (100) according to claim 1, wherein the open path of the contour (402) is substantially shaped, as viewed from a front of the side wall (121), as one of the following: curved; rounded; semicircular; semi-elliptical; U-shaped; horseshoe shaped, semi-oval; an arc of a partial circle; an arc of a partial ellipse; an arc of a partial oval; one third to three thirds of a circle; one third to three thirds of an oval; one third to three thirds of an ellipse; or a shape approximating an open-ended polygon.

4. The face soaking device (100) according to claim 1, wherein a cross-section of the receiving-channel (408) is substantially shaped as one of the following: curved; rounded; semicircular; semi-elliptical; U-shaped; horseshoe shaped, semi-oval; an arc of a partial circle; an arc of a partial ellipse; an arc of a partial oval; one third to three thirds of a circle; one third to three thirds of an oval; one third to three thirds of an ellipse; or a shape approximating an open-ended polygon.

5. The face soaking device (100) according to claim 1, wherein a direction that the receiving-channel (408) extends into the contour (402) is substantially parallel with a major plane of the side wall (121).

6. The face soaking device (100) according to claim 1, wherein a region comprising the neck-gasket-accommodator (400) and comprising where the neck-gasket-accommodator (400) joins the side wall (121); wherein this region, in cross-section, resembles an Arabic number "4".

7. The face soaking device (100) according to claim 1, wherein the vessel neck gasket (500) comprises a flexible member (505) and a carrier (511); wherein the flexible member (505) is flexible and substantially planar; wherein the carrier (511) is a third structural member that provides some rigidity to the flexible member (505); wherein the carrier (511) is attached to the flexible member (505); wherein both the carrier (511) and the flexible member (505) share the mating edge (501).

8. The face soaking device (100) according to claim 7, wherein the flexible member (505) is substantially elastomeric.

9. The face soaking device (100) according to claim 8, wherein when the at least portions of the mating-wall-edge (603) and the at least portions of the mating edge (501) are received into the receiving-channel (408), portions of the flexible member (505) within the receiving-channel (408) are compressed which contributes to forming the primary water tight seal.

10. The face soaking device (100) according to claim 7, wherein the flexible member (505) comprises a top edge (503) wherein at least a portion of the top edge (503) extends beyond the top rim (122) of the vessel (120) when the vessel neck gasket (500) is attached to the receiving-channel (408); wherein the top edge (503) and the mating edge (501) of the flexible member (505) define a closed shape for the flexible member (505).

11. The face soaking device (100) according to claim 7, wherein the carrier (511) is rigid to semi-rigid.

12. The face soaking device (100) according to claim 7, wherein the carrier (511) is shaped complimentary to a shape of the open path of the contour (402).

13. The face soaking device (100) according to claim 1, wherein the mating edge (501) is shaped complimentary to a shape of the receiving-channel (408).

14. The face soaking device (100) according to claim 1, wherein the mating-wall-edge (603) of the clamp (600) has a chamfer to facilitate insertion into the receiving-channel (408).

15. The face soaking device (100) according to claim 1, wherein the clamp (600) terminates in a finger-pull (811) that is engageble by a finger of a user to remove the clamp from the receiving-channel (408).

16. The face soaking device (100) according to claim 1, wherein the clamp (600) has a tab (815) that extends from the clamp (600) that is substantially perpendicular from a major plane of the side wall (121; wherein the tab (815) snaps onto a corner of the neck-gasket-accommodator (400).

17. The face soaking device (100) according to claim 1, wherein the clamp (600) is rigid to semi-rigid.

18. The face soaking device (100) according to claim 1, wherein a cross-section of the clamp (600) resembles: a closed polygon; a letter "f"; a letter "L"; a letter "J"; or a letter "C".

19. The face soaking device (100) according to claim 1, wherein the clamp (600) and the vessel neck gasket (500) are removable from the receiving-channel (408).

## Patentansprüche

1. Gesichtseinweichvorrichtung (100), umfassend:
ein Gefäß (120); und
Komponenten zum Bilden einer primären wasserdichten Abdichtung, umfassend:
eine Halsdichtungsaufnahme (400) zum Aufnehmen einer Gefäßhalsdichtung (500), die dazu ausgelegt ist, einen Teil des Halses eines Benutzers bequem aufzunehmen, wobei die Halsdichtungsaufnahme (400) ein erstes Bauteil ist, das sich an einer Seitenwand (121) des Gefäßes (120) befindet; wobei die Halsdichtungsaufnahme (400) eine Kontur (402) hat, die einem offenen Weg folgt, der unterhalb eines oberen Randes (122) des Gefäßes (120) liegt; wobei sich ein Aufnahmekanal (408) in die Kontur (402) erstreckt;
die Gefäßhalsdichtung (500), die eine Gegenkante (501) umfasst, wobei zumindest Abschnitte der Gegenkante (501) in den Aufnahmekanal (408) passen; und
eine Klemme (600), die ein zweites Bauteil mit einer Gegenwandkante (603) umfasst, wobei zumindest Abschnitte der Gegenwandkante (603) in den Aufnahmekanal (408) passen; und
wobei eine Breite eines Abschnitts der Klemme (600), eine Breite der Gefäßhalsdichtung (500) und eine Breite des Aufnahmekanals (408) derart bemessen sind, dass, wenn zumindest die Abschnitte der Gegenwandkante (603) und zumindest die Abschnitte der Gegenkante (501) in den Aufnahmekanal (408) aufgenommen werden, die primäre wasserdichte Abdichtung zwischen einem Abschnitt des Aufnahmekanals (408) und einigen Abschnitten der Gefäßhalsdichtung (500) gebildet wird, wobei ein Abschnitt der Gefäßhalsdichtung (500) zwischen der Klemme (600) und der Kontur (402) der Halsdichtungsaufnahme (400) eingefügt ist.

2. Gesichtseinweichvorrichtung (100) nach Anspruch 1, wobei der offene Weg der Kontur (402) durchgehend ist.

3. Gesichtseinweichvorrichtung (100) nach Anspruch 1, wobei der offene Weg der Kontur (402) im Wesentlichen, wenn von einer Vorderseite der Seitenwand (121) gesehen, gebildet ist wie eins der Folgenden: gebogen, gerundet, halbkreisförmig, halbelliptisch, U-förmig, hufeisenförmig, halboval, ein Bogen eines Halbkreises, ein Bogen einer Teilellipse, ein Bogen eines Teilovals, ein Drittel bis drei Drittel eines Kreises, ein Drittel bis drei Drittel eines Ovals, ein Drittel bis drei Drittel einer Ellipse oder eine Form, die sich einem Vieleck mit offenem Ende annähert.

4. Gesichtseinweichvorrichtung (100) nach Anspruch 1, wobei ein Querschnitt des Aufnahmekanals (408) im Wesentlichen geformt ist wie eins der Folgenden: gebogen, gerundet, halbkreisförmig, halbelliptisch, U-förmig, hufeisenförmig, halboval, ein Bogen eines Halbkreises, ein Bogen einer Teilellipse, ein Bogen eines Teilovals, ein Drittel bis drei Drittel eines Kreises, ein Drittel bis drei Drittel eines Ovals, ein Drittel bis drei Drittel einer Ellipse oder eine Form, die sich einem Vieleck mit offenem Ende annähert.

5. Gesichtseinweichvorrichtung (100) nach Anspruch 1, wobei eine Richtung, in die sich der Aufnahmekanal (408) in die Kontur (402) erstreckt, im Wesentlichen parallel zu einer Hauptebene der Seitenwand (121) ist.

6. Gesichtseinweichvorrichtung (100) nach Anspruch 1, wobei eine Region die Halsdichtungsaufnahme (400) umfasst und den Bereich, in dem sich die Halsdichtungsaufnahme (400) mit der Seitenwand (121) verbindet; wobei diese Region, im Querschnitt, an die arabische Ziffer "4" erinnert.

7. Gesichtseinweichvorrichtung (100) nach Anspruch 1, wobei die Gefäßhalsdichtung (500) ein flexibles Element (505) und einen Träger (511) umfasst; wobei das flexible Element (505) flexibel und im Wesentlichen plan ist; wobei der Träger (511) ein drittes Bauteil ist, das dem flexiblen Element (505) etwas Steifigkeit verleiht; wobei der Träger (511) an dem flexiblen Element (505) angebracht ist; wobei sowohl der Träger (511) als auch das flexible Element (505) die Gegenkante (501) teilen.

8. Gesichtseinweichvorrichtung (100) nach Anspruch 7, wobei der offene Weg der Kontur (505) durchgehend ist.

9. Gesichtseinweichvorrichtung (100) nach Anspruch 8, wobei, wenn mindestens die Abschnitte der Gegenwandkante (603) und mindestens die Abschnitte der Gegenkante (501) in den Aufnahmekanal (408) aufgenommen werden, Abschnitte des flexiblen Elements (505) in dem Aufnahmekanal (408) zusammengedrückt werden, was dazu beiträgt, die primäre wasserdichte Abdichtung zu bilden.

10. Gesichtseinweichvorrichtung (100) nach Anspruch 7, wobei das flexible Element (505) eine Oberkante (503) umfasst, wobei sich mindestens ein Abschnitt der Oberkante (503) über den oberen Rand (122) des Gefäßes (120) hinaus erstreckt, wenn die Gefäßhalsdichtung (500) an dem Aufnahmekanal (408) angebracht ist; wobei die Oberkante (503) und die Gegenkante (501) des flexiblen Elements (505) eine geschlossene Form für das flexible Element (505) definieren.

11. Gesichtseinweichvorrichtung (100) nach Anspruch 7, wobei der Träger (511) steif bis halbsteif ist.

12. Gesichtseinweichvorrichtung (100) nach Anspruch 7, wobei der Träger (511) komplementär zu einer Form des offenen Weges der Kontur (402) geformt ist.

13. Gesichtseinweichvorrichtung (100) nach Anspruch 1, wobei die Gegenkante (501) komplementär zu einer Form des Aufnahmekanals (408) geformt ist.

14. Gesichtseinweichvorrichtung (100) nach Anspruch 1, wobei die Gegenwandkante (603) der Klemme (600) eine Schräge hat, um den Einsatz in den Aufnahmekanal (408) zu erleichtern.

15. Gesichtseinweichvorrichtung (100) nach Anspruch 1, wobei die Klemme (600) in einem Fingerzug (811) mündet, der von einem Finger eines Benutzers gegriffen werden kann, um die Klemme aus dem Aufnahmekanal (408) zu entfernen.

16. Gesichtseinweichvorrichtung (100) nach Anspruch 1, wobei die Klemme (600) eine Lasche (815) hat, die sich von der Klemme (600) erstreckt, die im Wesentlichen senkrecht von einer Hauptebene der Seitenwand (121) ist, wobei die Lasche (815) an einer Ecke der Halsdichtungsaufnahme (400) einschnappt.

17. Gesichtseinweichvorrichtung (100) nach Anspruch 1, wobei die Klemme (600) steif bis halbsteif ist.

18. Gesichtseinweichvorrichtung (100) nach Anspruch 1, wobei ein Querschnitt der Klemme (600) erinnert an: ein geschlossenes Vieleck; ein "f", ein "L", ein "J" oder ein "C".

19. Gesichtseinweichvorrichtung (100) nach Anspruch 1, wobei die Klemme (600) und die Gefäßhalsdichtung (500) aus dem Aufnahmekanal (408) entfernbar sind.

## Revendications

1. Dispositif pour bain de visage (100) comprenant :
un récipient (120) ; et
des composants servant à former un joint étanche à l'eau principal comprenant :
un élément de réception de joint d'étanchéité de cou (400) destiné à recevoir un joint d'étanchéité de cou (500) du récipient qui est conçu pour qu'un utilisateur y place confortablement une partie de son cou, l'élément de réception de joint d'étanchéité de cou (400) étant un premier élément structurel situé sur une paroi latérale (121) du récipient (120) ; l'élément de réception de joint d'étanchéité de cou (400) comportant un contour (402) qui suit un passage ouvert situé sous un rebord supérieur (122) du récipient (120) ; un canal de réception (408) s'étendant dans le contour (402) ;
le joint d'étanchéité de cou (500) du récipient, comprenant un bord d'accouplement (501) ; au moins certaines parties du bord d'accouplement (501) s'adaptant à l'intérieur du canal de réception (408) ; et
une cale (600) qui est un deuxième élément structurel comportant un bord de paroi d'accouplement (603) ; au moins certaines parties du bord de paroi d'accouplement (603) s'adaptant à l'intérieur du canal de réception (408) ;
dans lequel une largeur d'une partie de la cale (600), une largeur du joint d'étanchéité de cou (500) du récipient et une largeur du canal de réception (408) sont d'une grandeur telle que, lorsque les au moins certaines parties du bord de paroi d'accouplement (603) et les au moins certaines parties du bord d'accouplement (501) sont reçues dans le canal de réception (408), le joint étanche à l'eau principal étant formé entre une partie du canal de réception (408) et certaines parties du joint d'étanchéité de cou (500) du récipient, une partie du joint d'étanchéité de cou (500) du récipient étant prise en sandwich entre la cale (600) et le contour (402) de l'élément de réception de joint d'étanchéité de cou (400) .

2. Dispositif pour bain de visage (100) selon la revendication 1, dans lequel le passage ouvert du contour (402) est continu.

3. Dispositif pour bain de visage (100) selon la revendication 1, dans lequel le passage ouvert du contour (402) présente sensiblement, en le regardant depuis un côté avant de la paroi latérale (121), l'une des formes suivantes : incurvée ; arrondie ; semi-circulaire ; semi-elliptique ; en U ; en fer à cheval, semi-ovale ; un arc d'un cercle partiel ; un arc d'une ellipse partielle ; un arc d'un ovale partiel ; un à trois tiers d'un cercle ; un à trois tiers d'un ovale ; un à trois tiers d'une ellipse ; ou une forme semblable à un polygone ouvert.

4. Dispositif pour bain de visage (100) selon la revendication 1, dans lequel une section transversale du canal de réception (408) présente sensiblement l'une des formes suivantes : incurvée ; arrondie ; semi-circulaire ; semi-elliptique ; en U ; en fer à cheval, semi-ovale ; un arc d'un cercle partiel ; un arc d'une ellipse partielle ; un arc d'un ovale partiel ; un à trois tiers d'un cercle ; un à trois tiers d'un ovale ; un à trois tiers d'une ellipse ; ou une forme semblable à un polygone ouvert.

5. Dispositif pour bain de visage (100) selon la revendication 1, dans lequel une direction dans laquelle s'étend le canal de réception (408) dans le contour (402) est sensiblement parallèle à un plan majeur de la paroi latérale (121).

6. Dispositif pour bain de visage (100) selon la revendication 1, dans lequel une région comprenant l'élément de réception de joint d'étanchéité de cou (400) et comprenant l'endroit où l'élément de réception de joint d'étanchéité de cou (400) est raccordé à la paroi latérale (121) ; dans lequel cette région, en section transversale, ressemble à un chiffre arabe « 4 ».

7. Dispositif pour bain de visage (100) selon la revendication 1, dans lequel le joint d'étanchéité de cou (500) du récipient comprend un élément flexible (505) et un support (511) ; dans lequel l'élément flexible (505) est flexible et sensiblement plat ; dans lequel le support (511) est un troisième élément structurel qui confère une certaine rigidité à l'élément flexible (505) ; dans lequel le support (511) est attaché à l'élément flexible (505) ; dans lequel le support (511) et l'élément flexible (505) partagent le bord d'accouplement (501).

8. Dispositif pour bain de visage (100) selon la revendication 7, dans lequel l'élément flexible (505) est essentiellement en élastomère.

9. Dispositif pour bain de visage (100) selon la revendication 8, dans lequel, lorsque les au moins certaines parties du bord de paroi d'accouplement (603) et les au moins certaines parties du bord d'accouplement (501) sont reçues dans le canal de réception (408), des parties de l'élément flexible (505) à l'intérieur du canal de réception (408) sont comprimées, ceci contribuant à former le joint étanche à l'eau principal.

10. Dispositif pour bain de visage (100) selon la revendication 7, dans lequel l'élément flexible (505) comprend un bord supérieur (503) ; dans lequel au moins une partie du bord supérieur (503) s'étend au-delà du rebord supérieur (122) du récipient (120) lorsque le joint d'étanchéité de cou (500) du récipient est attaché au canal de réception (408) ; dans lequel le bord supérieur (503) et le bord d'accouplement (501) de l'élément flexible (505) définissent une forme fermée pour l'élément flexible (505).

11. Dispositif pour bain de visage (100) selon la revendication 7, dans lequel le support (511) est rigide à semi-rigide.

12. Dispositif pour bain de visage (100) selon la revendication 7, dans lequel le support (511) présente une forme complémentaire d'une forme du passage ouvert du contour (402).

13. Dispositif pour bain de visage (100) selon la revendication 1, dans lequel le bord d'accouplement (501) présente une forme complémentaire d'une forme du canal de réception (408).

14. Dispositif pour bain de visage (100) selon la revendication 1, dans lequel le bord de paroi d'accouplement (603) de la cale (600) comporte un chanfrein pour faciliter l'insertion dans le canal de réception (408).

15. Dispositif pour bain de visage (100) selon la revendication 1, dans lequel la cale (600) se termine par une tirette (811) qu'un utilisateur peut manipuler avec son doigt afin de retirer la cale du canal de réception (408) .

16. Dispositif pour bain de visage (100) selon la revendication 1, dans lequel la cale (600) comporte une saillie (815) qui s'étend à partir de la cale (600) et qui est sensiblement perpendiculaire à un plan majeur de la paroi latérale (121) ; dans lequel la saillie (815) se met en prise élastiquement sur un coin de l'élément de réception de joint d'étanchéité de cou (400).

17. Dispositif pour bain de visage (100) selon la revendication 1, dans lequel la cale (600) est rigide à semi-rigide.

18. Dispositif pour bain de visage (100) selon la revendication 1, dans lequel une section transversale de la cale (600) ressemble à : un polygone fermé ; une lettre « f » ; une lettre « L » ; une lettre « J » ; ou une lettre « C ».

19. Dispositif pour bain de visage (100) selon la revendication 1, dans lequel la cale (600) et le joint d'étanchéité de cou (500) du récipient sont amovibles relativement au canal de réception (408).
